# EUROPEAN PATENT APPLICATION

(11) **EP 4 047 006 A1**
(43) Date of publication of application: **24.08.2022**
(21) Application number: 20876222.9
(22) Date of filing: 16.10.2020
(51) Int. Cl.: C07K 1/18, C07K 1/20, C07K 1/22, C07K 1/34, C07K 19/00, C12N 15/62

(54) **METHOD FOR PRODUCING FUSION PROTEIN OF SERUM ALBUMIN AND GROWTH HORMONE**

(30) Priority: 17.10.2019 JP 2019190600
(71) Applicant: JCR Pharmaceuticals Co., Ltd., Ashiya-shi, Hyogo 659-0021 (JP)
(72) Inventor: KAKIMOTO Shinji, Kobe-shi, Hyogo 651-2241 (JP); FUKUI, Tsuyoshi, Kobe-shi, Hyogo 651-2241 (JP); HATANO, Yukichi, Kobe-shi, Hyogo 651-2241 (JP); KOTANI Ayaka, Kobe-shi, Hyogo 651-2241 (JP); KATSUNO Ryota, Kobe-shi, Hyogo 651-2241 (JP); OKADA Yuko, Kobe-shi, Hyogo 651-2241 (JP)
(74) Representative: Balder IP Law, S.L.
(86) International application number: PCT/JP2020/039016
(87) International publication number: WO 2021/075526

(57) **Abstract**

Disclosed is a method for producing a fusion protein of human serum albumin and human growth hormone. The method comprises (a) a step of culturing a mammalian cell capable of producing the protein in a serum-free medium and causing the protein to be secreted into the culture fluid, (b) a step of collecting a culture supernatant by removing the mammalian cell from the culture fluid, and (c) a step of purifying the protein from the culture supernatant by using column chromatography using a material to which an antibody having an affinity for the protein is bound as a stationary phase, column chromatography using a material having an affinity for phosphate group as a stationary phase, cation exchange column chromatography, and size exclusion column chromatography.

## Description

### TECHNICAL FIELD

The present invention relates to a method for producing a fusion protein of serum albumin and a growth hormone, the fusion protein having a purity that allows the fusion protein to be used as it is as a medicine.

### BACKGROUND ART

Human growth hormone (hGH) is a protein secreted from the anterior pituitary gland under the control of the hypothalamus. hGH exhibits growth-promoting activity such as cartilage formation promotion and protein anabolism promotion, as well as body composition and lipid metabolism improving action. A child with low hGH secretion develops growth hormone deficiency dwarfism, which causes the child to exhibit short stature compared with a healthy child.

Preparations containing, as an active ingredient, a hGH (hGH preparations) having a molecular weight of about 22 kD produced as a recombinant protein by using hGH gene-introduced Escherichia coli have been clinically widely used as therapeutic agents for growth hormone deficiency dwarfism, short stature associated with Turner syndrome, Small-for-Gestational Age (SGA) dwarfism, dwarfism associated with Noonan syndrome, dwarfism caused by chronic renal failure, dwarfism associated with Prader-Willi syndrome, and dwarfism associated with cartilage dystrophy. hGH preparations are particularly effective in a case where these diseases are not accompanied by epiphyseal closure. hGH preparations are administered subcutaneously or intramuscularly to circulate in the blood and provide an effect of promoting the growth of a patient by the growth promoting activity of the hGH preparations. Furthermore, hGH preparations are widely clinically applied as a therapeutic agent for adult growth hormone deficiency. In patients with adult growth hormone deficiency, various abnormalities such as abnormal lipid metabolism are observed; however, when an hGH preparation is administered, the QOL of the patient is improved, such as that the patient's lipid metabolism is normalized. As an hGH preparation for growth hormone deficiency dwarfism, adult growth hormone deficiency, and the like, for example, GROWJECT (registered trademark) is available.

The half-life of hGH in blood plasma is considered to be less than 20 minutes, and hGH administered to a patient rapidly disappears from the blood. Therefore, in order to have the efficacy of hGH substantially exhibited in a patient, it is necessary to administer hGH to the patient intramuscularly three times a week, or subcutaneously daily. Such frequent administration poses a burden on patients. Therefore, when it is possible to reduce the frequency of administration of hGH to a patient by increasing the stability of hGH in blood plasma and prolonging the half-life, the burden on the patient can be reduced, which is preferable.

Human serum albumin (HSA) is a protein whose mature form consists of 585 amino acids. When one simply refers to human serum albumin, it means this mature form. HSA is a component that is most abundant among plasma proteins, and the half-life in blood plasma is as long as 14 to 20 days. HSA contributes to the regulation of plasma osmotic pressure and has a function of binding to cations, fatty acids, hormones, bilirubin, and other endogenous substances in the blood as well as exogenous substances such as drugs and transporting them. Generally, a substance bound to HSA is less likely to be taken up by organs and can circulate in the blood for a long period of time.

It is known that there are some of natural variants of human serum albumin (HSA). Human serum albumin Redhill is one of them (Non-Patent Document 1 and 2). Human serum albumin Redhill is different, as compared to the amino acid sequence of the above-described normal human serum albumin consisting of 585 amino acids, from the viewpoint that the 320^{th} amino acid residue from the N-terminal side of human serum albumin Redhill is threonine instead of alanine and one arginine residue is added to the N-terminus thereof, so that human serum albumin Redhill consists of 586 amino acids. Due to the change from alanine to threonine, a sequence represented by Asn-Tyr-Thr is produced in the amino acid sequence of albumin Redhill, and the Asn (asparagine) residue in this sequence is N-linked glycosidated. Therefore, albumin Redhill is observed to have a molecular weight that is larger by about 2.5 kDa compared with the above-described normal human serum albumin.

Methods of increasing the stability of hGH in blood plasma by linking hGH to HSA have been reported (Patent Document 1 to 8). A protein obtained by linking HSA to hGH is produced as a recombinant protein in a medium or in cells, by producing a transformed cell into which an expression vector incorporating a DNA in which a gene encoding hGH and a gene encoding HSA are linked in-frame is introduced and culturing this cell.

### CITATION LIST

### PATENT DOCUMENTS

Patent Document 1: WO 2017/154869
Patent Document 2: WO 2017/159540
Patent Document 3: JP 2003-530838 A
Patent Document 4: JP 2005-514060 A
Patent Document 5: JP 2000-502901 A
Patent Document 6: JP 2008-518615 A
Patent Document 7: JP 2013-501036 A
Patent Document 8: JP 2013-518038 A

### DISCLOSURE OF THE INVENTION

### PROBLEM TO BE SOLVED BY THE INVENTION

Under the above-described circumstances, an object of the present invention is to provide a method for producing a fusion protein of human albumin and human growth hormone, the fusion protein having a purity that allows the fusion protein to be used as it is as a medicine, and a medicine containing the fusion protein as an active ingredient.

### MEANS FOR SOLVING PROBLEM

The inventors of the present invention repeatedly conducted a thorough investigation in a study directed to the above-described object, and as a result, the inventors found a method for efficiently producing a compound (human serum albumin mutant-hGH fusion protein) obtained by linking a mutant (human serum albumin mutant) comprising, as compared with normal human serum albumin consisting of 585 amino acids, an amino acid sequence in which arginine as the 320^{th} amino acid residue from the N-terminus of normal human serum albumin has been substituted with threonine, to human growth hormone (hGH), the compound having a purity that allows the compound to be used as it is as a medicine. Thus, the inventors completed the present invention. That is, the present invention includes the following.
1. A method for producing a fusion protein of human serum albumin and human growth hormone,
   the method including:
   (a) a step of culturing a mammalian cell capable of producing the protein in a serum-free medium and causing the protein to be secreted into the culture fluid;
   (b) a step of collecting a culture supernatant by removing the mammalian cell from the culture fluid obtained in the step (a); and
   (c) a step of purifying the protein from the culture supernatant obtained in the step (b), by using column chromatography using a material to which an antibody having an affinity for the protein is bound as a stationary phase, column chromatography using a material having an affinity for phosphate group as a stationary phase, cation exchange column chromatography, and size exclusion column chromatography.
2. The production method according to the above-described item 1, wherein in the step (c), column chromatography using a material in which an antibody having an affinity for the protein is bound as a stationary phase, column chromatography using a material having an affinity for phosphate group as a stationary phase, cation exchange column chromatography, and size exclusion column chromatography are used in this order.
3. The production method according to the above-described item 1 or 2, wherein the antibody having an affinity for the protein has an affinity to human serum albumin or human growth hormone.
4. The production method according to the above-described item 1 or 2, wherein the antibody having an affinity for the fusion protein has an affinity for human growth hormone.
5. The production method according to any one of the above-described items 1 to 4, wherein the material having an affinity for the phosphate group is either fluoroapatite or hydroxyapatite.
6. The production method according to any one of the above-described items 1 to 4, wherein the material having an affinity for the phosphate group is hydroxyapatite.
7. The production method according to any one of the above-described items 1 to 6, wherein a cation exchanger used for the cation exchange column chromatography is a weak cation exchanger.
8. The production method according to the above-described item 7, wherein the weak cation exchanger retains selectivity based on both hydrophobic interaction and hydrophobic bond formation.
9. The production method according to any one of the above-described items 1 to 8, further including a step for inactivating viruses.
10. The production method according to the above-described item 9, wherein an eluate of the protein obtained by column chromatography using a material to which an antibody having an affinity for the protein is bound as a stationary phase, is subjected to inactivation of viruses.
11. The production method according to the above-described item 9, wherein an eluate of the protein obtained by the size exclusion column chromatography is subjected to inactivation of viruses.
12. The production method according to any one of the above-described items 1 to 8, wherein the production method includes two times of the step for inactivating viruses.
13. The production method according to the above-described item 12, wherein an eluate of the protein obtained by column chromatography using a material to which an antibody having an affinity for the protein is bound as a stationary phase, and an eluate of the protein obtained by the size exclusion column chromatography are subjected to inactivation of viruses.
14. The production method according to the above-described item 12 or 13, wherein one time of the step for inactivating viruses is carried out by a method of inactivating viruses by adding a nonionic surfactant to a solution containing HSA-hGH fusion protein, and the other one time is carried out by a method of passing the solution containing HSA-hGH fusion protein through a filtration membrane.
15. The production method according to any one of the above-described items 1 to 14, wherein the protein is formed such that human serum albumin is linked by a peptide bond to the C-terminal side of human growth hormone directly or via a linker sequence.
16. The production method according to the above-described item 15, wherein the linker sequence includes an amino acid sequence selected from the group consisting of one glycine, one serine, amino acid sequence (Gly-Ser), amino acid sequence (Gly-Gly-Ser), and an amino acid sequence consisting of one to ten consecutive sequences of these amino acid sequences.
17. The production method according to the above-described item 16, wherein the linker sequence has an amino acid sequence represented by amino acid sequence (Gly-Ser).
18. The production method according to any one of the above-described items 1 to 14, wherein the amino acid sequence of the protein has an identity of 85% or higher with an amino acid sequence set forth in SEQ ID NO:11.
19. The production method according to any one of the above-described items 1 to 14, wherein the amino acid sequence of the protein has an identity of 95% or higher with the amino acid sequence set forth in SEQ ID NO:11.
20. The production method according to any one of the above-described items 1 to 14, wherein the amino acid sequence of the protein has the amino acid sequence set forth in SEQ ID NO:11.

### EFFECT OF THE INVENTION

According to the present invention, for example, a fusion protein of serum albumin and growth hormone purified to the extent that the fusion protein can be distributed in the market as a medicine, can be efficiently produced.

### BRIEF DESCRIPTION OF DRAWINGS

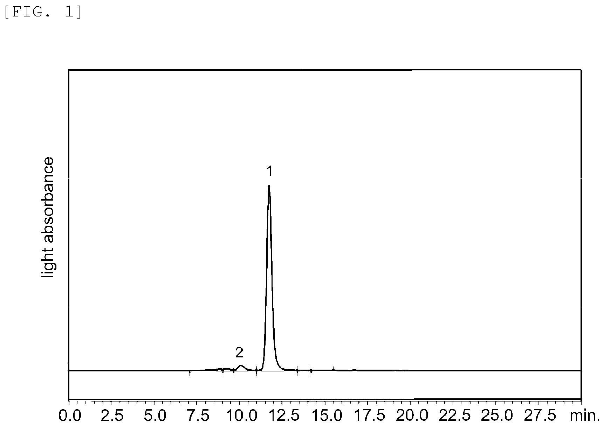

Fig. 1 shows a SE-HPLC chart of 22KhGH-mHSA purified product. The axis of ordinate represents the light absorbance (arbitrary unit) at 215 nm, and the axis of abscissa represents the retention time (minutes). In the drawing, peak 1 corresponds to a monomer of the 22KhGH-mHSA purified product, and peak 2 corresponds to a polymer of the 22KhGH-mHSA purified product.

### MODE(S) FOR CARRYING OUT THE INVENTION

According to the present specification, when the term "human serum albumin" or "HSA" is simply used, this also includes, in addition to the normal wild-type human serum albumin consisting of 585 amino acid residues shown in SEQ ID NO:1, a mutant of HSA corresponding to an amino acid sequence set forth in SEQ ID NO:1, in which one amino acid residue or a plurality of amino acid residues have been substituted, deleted, and/or added (according to the present specification, the "addition" of an amino acid residue means addition of a residue at a terminus or the inner part of the sequence), without particular discrimination as long as the mutant has functions as normal wild-type human serum albumin, such as a function of binding to endogenous substances in the blood and exogenous substances such as drugs and transporting them. In the case of substituting an amino acid residue with another amino acid residue, the number of amino acid residues to be substituted is preferably 1 to 10, more preferably 1 to 5, and even more preferably 1 to 3. In the case of deleting an amino acid residue, the number of amino acid residues to be deleted is preferably 1 to 10, more preferably 1 to 5, and even more preferably 1 to 3. For example, a mutant consisting of 584 amino acid residues, in which an amino acid residue at the N-terminus or C-terminus of the amino acid sequence set forth in SEQ ID NO:1 has been deleted, is also included in human serum albumin. Furthermore, substitution and deletion of these amino acid residues may be combined. Furthermore, a sequence in which one amino acid residue or a plurality of amino acid residues have been added into the amino acid sequence of normal wild-type HSA or a mutant thereof, or at the N-terminal side or C-terminal side of the amino acid sequence, is also acceptable. The number of amino acid residues to be added at this time is preferably 1 to 10, more preferably 1 to 5, and even more preferably 1 to 3.

Regarding a mutant of HSA into which a combination of at least two kinds of mutations among these three kinds of mutations, substitution, deletion and addition of amino acids, has been introduced, it is preferable that the mutant has an amino acid sequence obtained by performing deletion of 0 to 10 amino acid residues, substitution of 0 to 10 amino acid residues with other amino acid residues, and addition of 0 to 10 amino acid residues, with respect to the amino acid sequence set forth in SEQ ID NO:1. More preferably, the number of those amino acid residues to be subjected to each of those deletion, substitution, and/or addition with respect to the amino acid sequence set forth in SEQ ID NO:1 is preferably 5 or less, and more preferably 3 or less.

According to the present invention, the term "human serum albumin Redhill" (HSA-Redhill) means a variant of human serum albumin, which consists of 586 amino acid residues as set forth in SEQ ID NO:2. Human serum albumin Redhill corresponds to a product in which, with regard to the amino acid sequence of wild-type human serum albumin consisting of 585 amino acids as set forth in SEQ ID NO:1, the 320^{th} amino acid residue from the N-terminus is threonine instead of alanine, and one arginine residue has been added to the N-terminus. Due to this substitution of alanine with threonine, a sequence portion represented by Asn-Tyr-Thr is produced in the amino acid sequence of albumin Redhill, and the Asn (asparagine) residue in this sequence portion is N-linked glycosidated. For this reason, albumin Redhill is observed to have a molecular weight larger by about 2.5 kDa compared with normal wild-type albumin (SEQ ID NO:1).

According to the present invention, the term "human serum albumin mutant" (HSA mutant) means the above-mentioned mutant for normal wild-type HSA (SEQ ID NO:1), provided that the mutant is other than the variant set forth in SEQ ID NO:2 (HSA-Redhill). A preferred HSA mutant according to the present invention includes, in addition to an amino acid sequence set forth in SEQ ID NO:3 in which the 320^{th} alanine from the N-terminus of the amino acid sequence of wild-type HSA has been substituted with threonine, an amino acid sequence in which one amino acid residue or a plurality of amino acid residues in the amino acid sequence set forth in SEQ ID NO:3 have been substituted with other amino acid residues, deleted, or added, provided that the 318^{th} asparagine residue and the 320^{th} threonine residue from the N-terminus of the amino acid sequence set forth in SEQ ID NO:3 are conserved in a state of being linked by a peptide bond, with a single amino acid residue (X) other than proline interposed between those two residues, as long as the amino acid sequence has the functions of normal wild-type human serum albumin, such as a function of binding to endogenous substances in the blood and exogenous substances such as drugs and transporting them. When an amino acid residue in the amino acid sequence is substituted with another amino acid residue, the number of amino acid residues to be substituted is preferably 1 to 10, more preferably 1 to 5, and even more preferably 1 to 3. In the case of deleting an amino acid residue, the number of amino acid residues to be deleted is preferably 1 to 10, more preferably 1 to 5, and even more preferably 1 to 3. For example, the HSA mutant may also be a mutant consisting of 584 amino acid residues, in which an amino acid residue at the N-terminus or C-terminus of the amino acid sequence set forth in SEQ ID NO:3 has been deleted. Furthermore, a combination of these substitutions and deletions of amino acid residues is also acceptable. In addition, products obtained by adding one amino acid residue or a plurality of amino acid residues into the amino acid sequences of those mutants or to the N-terminal side or the C-terminal side of the amino acid sequences, are also acceptable. That is, the HSA mutant may be a product in which a combination of at least two kinds of mutations among the three kinds of mutations, substitution, deletion, and addition of amino acids, has been introduced into the amino acid sequence set forth in SEQ ID NO:3, and in which deletion of 0 to 10 amino acid residues, substitution of 0 to 10 amino acid residues with other amino acid residues, and addition of 0 to 10 amino acid residues have been carried out. However, the 318^{th} to 320^{th} amino acid residues from the N-terminus of the amino acid sequence set forth in SEQ ID NO: 3 must be asparagine-X-threonine ("X" is an amino acid residue other than proline), and the sequence is asparagine-tyrosine-threonine. Incidentally, the human serum albumin mutant is included in human serum albumin as long as the mutant retains the functions as human serum albumin. Here, the amino acid sequence of the human serum albumin mutant preferably has an identity of 85% or higher, more preferably an identity of 90% or higher, and even more preferably an identity of 95% or higher, with the amino acid sequence of normal wild-type HSA set forth in SEQ ID NO:1.

According to the present invention, the position of each mutation and the form thereof (deletion, substitution, or addition) in each of various HSA mutants as compared with normal wild-type HSA can be easily checked by checking the alignment of the amino acid sequences of the two HSAs. According to the present invention, the identity between the amino acid sequence of wild-type HSA and the amino acid sequence of HSA to which mutation has been applied, can be easily calculated by using well-known homology calculation algorithms. Examples of such algorithms include BLAST (Altschul SF. J Mol. Biol., 215, 403-10 (1990)), the similarity search method of Pearson and Lipman (Proc. Natl. Acad. Sci., USA, 85, 2444 (1988)), and the local homology algorithm of Smith and Waterman (Adv. Appl. Math., 2, 482-9 (1981)).

With regard to the substitution of an amino acid in the above-described amino acid sequence of HSA with another amino acid, for example, amino acids classified as the same groups, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxy group (Ser, Thr), and amino acids having a small side chain (Gly, Ala, Ser, Thr, Met), may be mentioned. Substitution with such a similar amino acid is not expected to bring significant changes in the functions of HSA (that is, conservative amino acid substitution).

A human serum albumin mutant linked to human growth hormone (a typical example of HSA mutants) in the Examples that will be described below is different from the amino acid sequence of wild-type human serum albumin (SEQ ID NO:1) consisting of 585 amino acids, only in that the 320^{th} amino acid residue from the N-terminus is threonine instead of alanine (SEQ ID NO:3). Due to this difference, in this HSA mutant (referred to as "HSA(A320T)"), a sequence portion represented by Asn-Tyr-Thr is produced in the amino acid sequence, and the Asn (asparagine) residue in this sequence portion can be N-linked glycosidated.

According to the present specification, the term "fusion protein of human serum albumin and human growth hormone" or "human serum albumin-hGH fusion protein (HSA-hGH fusion protein)" means growth hormone to which HSA is linked, and this is a compound obtainable by linking the polypeptides each having the respective amino acid sequences of the two. Here, when it is said that those polypeptides are "linked", not only the case of using a direct peptide bond between the N-terminus of one polypeptide and the C-terminus of the other polypeptide, but also the case of indirectly linking the two polypeptides via a linker, are also included. Particularly, when human serum albumin is a human serum albumin mutant, this is referred to as "fusion protein of a human serum albumin mutant and human growth hormone" or "human serum albumin mutant-hGH fusion protein (HSA mutant-hGH fusion protein)". That is, the human serum albumin mutant-hGH fusion protein is included in the human serum albumin-hGH fusion protein.

Here, the "linker" is a structural part that links the above-described two polypeptides by covalent bonding between the two polypeptides, and the linker is derived from neither of the ends of HSA (including an HSA mutant) and growth hormone as the bonding partner. The linker can be a single amino acid residue or a peptide chain moiety (peptide linker) consisting of two or more amino acid residues, both being linked by a peptide bond to the two polypeptides, and these linkers each consisting of one or more amino acid residues are comprehensively referred to as "peptide linker" in the present specification. Furthermore, according to the present specification, when it is said that HSA and growth hormone are linked "through a peptide bond", this includes the case where the two are directly linked by a peptide bond, and the case where the two are linked by linkage to a peptide linker. Incidentally, in the present specification, when HSA and growth hormone are linked directly or through a peptide linker, the compound "human serum albumin-hGH fusion protein (HSA-hGH fusion protein)" can also be said to be "human serum albumin-fused hGH (HSA-fused hGH)". The same also applies to human serum albumin mutant-hGH fusion protein (HSA mutant-hGH fusion protein).

According to the present invention, when HSA and growth hormone are linked via a peptide linker, the linker is preferably composed of 1 to 50, more preferably 1 to 17, even more preferably 1 to 10, and still more preferably 1 to 6, amino acid residues, and for example, the linker composed of 2 to 17, 2 to 10, 10 to 40, 20 to 34, 23 to 31, or 25 to 29, amino acids, while for example, the linker is composed of only one amino acid residue or composed of 2, 3, 5, 6, or 20 amino acid residues. The amino acid residue or amino acid sequence constituting the peptide linker is not limited as long as the HSA moiety linked by the peptide linker retains the functions as HSA, and the growth hormone moiety can also exhibit the physiological activity of growth hormone under physiological conditions; however, it is preferable that the peptide linker is composed of glycine and serine. Suitable examples of the peptide linker include peptide linkers consisting of Gly-Ser, Gly-Gly-Ser, Gly-Gly-Gly-Gly-Ser (SEQ ID NO:4), Gly-Gly-Gly-Gly-Gly-Ser (SEQ ID NO:5), and Ser-Gly-Gly-Gly-Gly-Gly (SEQ ID NO:6); and peptide linkers including these amino acid sequences. A peptide linker having a sequence including any one kind of these amino acid sequences, which is repeated 2 to 10 times or 2 to 5 times consecutively, can also be suitably used as the peptide linker, and a peptide linker having a sequence including any two or more kinds of these amino acid sequences in combination, which are repeated 1 to 10 times or 2 to 5 times consecutively, can also be suitably used as the peptide linker. A suitable example of a peptide linker having any two or more kinds of these amino acid sequences in combination may be a peptide linker that includes an amino acid sequence having a total of 20 amino acids, composed of the amino acid sequence Gly-Ser followed by three consecutive repetitions of the amino acid sequence Gly-Gly-Gly-Gly-Gly-Ser (SEQ ID NO:5).

Regarding a method of linking two different polypeptides, for example, a method of producing an expression vector having a DNA incorporated therein, the DNA being a DNA in which a gene encoding one of the polypeptides is linked in-frame downstream of a gene encoding the other polypeptide, and culturing a host cell that has been transformed by using this expression vector to express the expression vector as a recombinant fusion protein, is general and can be utilized in the present invention.

When the HSA-hGH fusion protein is produced by expressing the fusion protein as a recombinant in a transformed cell, a fusion protein in the form in which a polypeptide including the amino acid sequence of growth hormone is linked to either the N-terminus or the C-terminus of a polypeptide including the amino acid sequence of HSA is obtained. An HSA-hGH fusion protein produced by using the gene recombination technology is particularly referred to as recombinant HSA-hGH fusion protein.

When the polypeptide including the amino acid sequence of growth hormone is linked to the N-terminal side of the polypeptide including the amino acid sequence of HSA, an expression vector having a DNA incorporated therein, the DNA being a DNA in which a gene encoding the polypeptide that includes the amino acid sequence of HSA is linked in-frame downstream of a gene encoding the polypeptide that includes the amino acid sequence of growth hormone, is used. When the two polypeptides are indirectly linked through a peptide linker, a DNA sequence encoding the linker is inserted in-frame between the genes encoding the two polypeptides.

When the polypeptide including the amino acid sequence of growth hormone is linked to the C-terminal side of the polypeptide including the amino acid sequence of HSA, an expression vector having a DNA incorporated therein, the DNA being a DNA in which a gene encoding the polypeptide that includes the amino acid sequence of HSA is linked in-frame upstream of a gene encoding the polypeptide that includes the amino acid sequence of growth hormone, is used. When the two polypeptides are indirectly linked through a peptide linker, a DNA sequence encoding the linker is inserted in-frame between the genes encoding the two polypeptides.

In order to produce the HSA-hGH fusion protein in a host cell, an expression vector in which a DNA encoding any one of those is incorporated is introduced into the host cell. The host cell that can be used for this purpose is not particularly limited as long as the host cell can express the HSA-hGH fusion protein when such an expression vector is introduced therein, and the host cell may be any one of eukaryotic cells such as a mammalian cell, yeast, a plant cell, and an insect cell; and prokaryotic cells such as Escherichia coli and Bacillus subtilis; however, a mammalian cell is particularly suitable. However, the host cell in the case of expressing as a sugar chain-modified protein is selected from eukaryotic cells such as a mammalian cell, yeast, a plant cell, and an insect cell. The Asn residue of a sequence portion represented by Asn-Tyr-Thr, which is produced when the 320^{th} amino acid residue of normal wild-type HSA is converted to threonine, or the Asn residue in a sequence represented by Asn-X-Thr ("X" is an amino acid residue other than proline) is N-linked glycosidated by performing the expression of the HSA-hGH fusion protein in a eukaryotic cell.

When a mammalian cell is used as the host cell, the type of this mammalian cell is not particularly limited; however, a cell derived from human, mouse, or Chinese hamster is preferred, and Chinese hamster ovarian cell-derived CHO cell, or mouse myeloma-derived NS/0 cell is preferred. Furthermore, regarding the expression vector that is used at this time in order to incorporate and express a DNA fragment encoding the HSA-hGH fusion protein, any expression vector that brings expression of the gene when introduced into a mammalian cell can be used without particular limitation. The gene incorporated into the expression vector is disposed downstream of a DNA sequence (gene expression control site) capable of regulating the frequency of gene transcription in a mammalian cell. Examples of the gene expression control site that can be used for the present invention include a cytomegalovirus-derived promoter, an SV40 early promoter, a human elongation factor-la (EF-1α) promoter, and a human ubiquitin C promoter.

Mammalian cells into which such an expression vector has been introduced will express the protein incorporated in the expression vector; however, the amount of expression thereof may vary from individual cell to individual cell and may not be uniform. Therefore, in order to efficiently produce the HSA-hGH fusion protein, a step of selecting these cells having high expression levels from the mammalian cells into which the expression vector has been introduced is necessary. In order to carry out this selection step, a gene working as a selectable marker is incorporated into the expression vector.

A most general selectable marker is an enzyme that degrades drugs such as puromycin and neomycin (drug resistance marker). Mammalian cells usually die out in the presence of these drugs at or above certain concentrations. However, mammalian cells into which an expression vector having a drug resistance marker gene incorporated therein has been introduced, can survive even in the presence of the above-described drugs because the drugs can be detoxified or attenuated by the expressed drug resistance marker. When an expression vector having a drug resistance marker incorporated therein as the selectable marker is introduced into mammalian cells, and culture is continued in a selective medium containing the drug corresponding to the drug resistance marker, for example, while gradually increasing the concentration of the drug, a cell that can proliferate even in the presence of a higher concentration of the drug is obtained. In the cells selected in this manner, the amount of expression of a gene encoding a target protein, which has been incorporated into an expression vector, is generally increased together with the drug resistance marker, and as a result, cells having a high expression level of the protein are selected.

Furthermore, glutamine synthetase (GS) can also be used as the selectable marker. Glutamine synthetase is an enzyme that synthesizes glutamine from glutamic acid and ammonia. When mammalian cells are cultured in a selective medium that contains an inhibitor of glutamine synthetase, for example, L-methionine sulfoximine (MSX), and does not contain glutamine, the cells usually die out. However, when an expression vector in which glutamine synthetase has been incorporated as the selectable marker is introduced into mammalian cells, the expression level of glutamine synthetase is increased in the cells, and therefore, the cells can proliferate even in the presence of a higher concentration of MSX. At this time, when culture is continued while gradually increasing the concentration of MSX, cells capable of proliferating even in the presence of a higher concentration of MSX are obtained. In the cells selected in this manner, the amount of expression of a gene encoding a target protein, which has been incorporated into an expression vector, is generally increased together with the glutamine synthetase, and as a result, cells having a high expression level of the protein are selected.

Furthermore, dihydrofolate reductase (DHFR) can also be used as the selectable marker. When DHFR is used as the selectable marker, mammalian cells into which the expression vector has been introduced are cultured in a selective medium containing a DHFR inhibitor such as methotrexate or aminopterin. When culture is continued while gradually increasing the concentration of the DHFR inhibitor, cells capable of proliferating even in the presence of a higher concentration of the DHFR inhibitor are obtained. It is preferable that the selective medium to be used at this time does not contain hypoxanthine and thymidine. In the cells selected in this manner, the amount of expression of a gene encoding a target protein, which has been incorporated into an expression vector, is generally increased together with DHFR, and as a result, cells having a high expression level of the protein are selected.

Expression vectors in which glutamine synthetase (GS) as a selectable marker is disposed downstream of a gene encoding a target protein, with an internal ribosome entry site (IRES) interposed therebetween, are known (WO 2012/063799 and WO 2013/161958). The expression vectors described in these documents can be particularly suitably used for the production of the HSA-hGH fusion protein.

For example, an expression vector for expressing a target protein, the expression vector including a first gene expression control site, a gene encoding the protein downstream thereof, an internal ribosome entry site further downstream, and a gene encoding glutamine synthetase even further downstream, and the expression vector further including dihydrofolate reductase gene or a drug resistance gene downstream of the first gene expression control site or a second gene expression control site different from this first gene expression control site, can be suitably used for the production of the HSA-hGH fusion protein. With regard to this expression vector, as the first gene expression control site or the second gene expression control site, a cytomegalovirus-derived promoter, an SV40 early promoter, a human elongation factor-la promoter (hEF-1α promoter), and a human ubiquitin C promoter are suitably used; however, a hEF-1α promoter is particularly suitable.

Furthermore, regarding the internal ribosome entry site, such a site derived from a 5'-untranslated region of the genome of a virus selected from the group consisting of a virus of the family Picornavirus, foot-and-mouth disease virus, hepatitis A virus, hepatitis C virus, a coronavirus, bovine enterovirus, Theiler's murine encephalomyelitis virus, and Coxsackie B virus, or a gene selected from the group consisting of human immunoglobulin heavy chain binding protein, Drosophila Antennapedia gene, and Drosophila Ultrabithorax gene, is suitably used; however, an internal ribosome entry site derived from the 5'-untranslated region of the mouse encephalomyelitis virus genome is particularly suitable. When an internal ribosome entry site derived from the 5'-untranslated region of the mouse encephalomyelitis virus genome is used, the wild-type internal ribosome entry site as well as an internal ribosome entry site in which some of a plurality of start codons included in the wild-type internal ribosome entry site have been destroyed, can also be suitably used. Furthermore, a drug resistance gene that is suitably used for this expression vector is preferably the puromycin or neomycin resistance gene, and more preferably the puromycin resistance gene.

Furthermore, for example, an expression vector for expressing a target protein, the expression vector including a human elongation factor-la promoter, a gene encoding the protein downstream thereof, an internal ribosome entry site derived from a 5'-untranslated region of the mouse encephalomyelitis virus genome further downstream, and a gene encoding glutamine synthetase even further downstream, and further including another gene expression control site and dihydrofolate reductase gene downstream thereof, in which the internal ribosome entry site is an internal ribosome entry site in which some of a plurality of start codons included in the wild-type internal ribosome entry site have been destroyed, can be suitably used for the production of the HSA-hGH fusion protein. Examples of such an expression vector include the expression vector described in WO 2013/161958.

Furthermore, for example, an expression vector for expressing a target protein, the expression vector including a human elongation factor-la promoter, a gene encoding the protein downstream thereof, an internal ribosome entry site derived from a 5'-untranslated region of the mouse encephalomyelitis virus genome further downstream, and a gene encoding glutamine synthetase even further downstream, and further including another gene expression control site and a drug resistance gene downstream thereof, in which the internal ribosome entry site is an internal ribosome entry site in which some of a plurality of start codons included in the wild-type internal ribosome entry site have been destroyed, can be suitably used for the production of the HSA-hGH fusion protein. Examples of such an expression vector include pE-mIRES-GS-puro described in WO 2012/063799 and pE-mIRES-GS-mNeo described in WO 2013/161958.

At the 3'-terminus of the internal ribosome entry site derived from the 5'-untranslated region of the wild-type mouse encephalomyelitis virus genome, there are three start codons (ATG), and the sequence portion including the three start codons is set forth in SEQ ID NO:7 (5'-ATGataatATGgccacaaccATG-3': ATG of the start codon is indicated with capital letters). As an internal ribosome entry site in which some of the start codons in this sequence portion have been destroyed, for example, there is a sequence set forth in SEQ ID NO:8 (5'-atgataagcttgccacaaccatg-3'), and the above-described pE-mIRES-GS-puro and pE-mIRES-GS-mNeo are expression vectors having IRES including the sequence set forth in SEQ ID NO:8.

According to the present invention, mammalian cells into which an expression vector having a DNA fragment encoding the HSA-hGH fusion protein has been introduced, are selectively cultured in a selectable medium in order to select cells having high expression levels of these proteins.

When DHFR is used as the selectable marker in selective culture, the concentration of a DHFR inhibitor included in the selective medium is increased stepwise. When the DHFR inhibitor is methotrexate, the maximum concentration thereof is preferably 0.25 to 5 µM, more preferably 0.5 to 1.5 µM, and even more preferably about 1.0 µM.

When GS is used as the selectable marker, the concentration of a GS inhibitor included in the selective medium is increased stepwise. When the GS inhibitor is MSX, the maximum concentration thereof is preferably 10 to 1000 µM or the like, and for example, the maximum concentration is 20 to 500 µM, 20 to 80 µM, or 20 to 30 µM. Furthermore, at this time, a medium that does not contain glutamine is generally used as the selective medium.

When an enzyme that degrades puromycin is used as a selectable marker, the maximum concentration of puromycin included in the selectable marker is preferably 3 to 30 µg/mL, more preferably 5 to 20 µg/mL, and even more preferably about 10 µg/mL.

When an enzyme that degrades neomycin is used as a selectable marker, the maximum concentration of G418 included in the selective medium is preferably 0.1 to 2 mg/mL, more preferably 0.5 to 1.5 mg/mL, and even more preferably about 1 mg/mL.

Furthermore, regarding the medium for culturing mammalian cells, any medium capable of culturing and proliferating mammalian cells can be used without particular limitation, together with a medium that is used for selective culture and a medium that is used for producing a recombinant protein (medium for recombinant protein production), which will be described below; however, a serum-free medium is preferably used. Since HSA has a property of adsorbing the components included in blood serum, when HSA is produced by using a medium containing blood serum, HSA that has adsorbed impurities in the blood serum is obtained, and therefore, it is necessary to remove these impurities in a subsequent step.

According to the present invention, the HSA-hGH fusion protein is particularly a fusion protein obtainable by culturing cells expressing these in a serum-free medium. Since the amount of adsorption of impurities to the HSA-hGH fusion protein can be reduced by using a serum-free medium, the subsequent purification steps can be simplified.

Cells selected by selective culture and having a high expression level of the HSA-hGH fusion protein are used for the production of the HSA-hGH fusion protein (HSA-hGH fusion protein producing cells). The production of the HSA-hGH fusion protein is carried out by culturing the HSA-hGH fusion protein producing cells in a medium for HSA-hGH fusion protein production. This culture is referred to as productive culture.

According to the present invention, as the serum-free medium to be used as the medium for HSA-hGH fusion protein production, for example, a medium containing 3 to 700 mg/L of amino acids, 0.001 to 50 mg/L of vitamins, 0.3 to 10 g/L of polysaccharides, 0.1 to 10000 mg/L of inorganic salts, 0.001 to 0.1 mg/L of trace elements, 0.1 to 50 mg/L of nucleoside, 0.001 to 10 mg/L of fatty acids, 0.01 to 1 mg/L of biotin, 0.1 to 20 µg/L of hydrocortisone, 0.1 to 20 mg/L of insulin, 0.1 to 10 mg/L of vitamin B12, 0.01 to 1 mg/L of putrescine, 10 to 500 mg/L of sodium pyruvate, and water-soluble iron compounds is suitably used. If desired, thymidine, hypoxanthine, a conventional pH indicator, an antibiotic substance, and the like may also be added to the medium.

Regarding the serum-free medium to be used as the medium for HSA-hGH fusion protein production, DMEM/F12 medium (mixed medium of DMEM and F12) may be used as a basic medium, and these media are respectively well known to those ordinarily skilled in the art. Furthermore, DMEM(HG)HAM modified (R5) medium, which includes sodium hydrogen carbonate, L-glutamine, D-glucose, insulin, sodium selenite, diaminobutane, hydrocortisone, iron(II) sulfate, asparagine, aspartic acid, serine, and polyvinyl alcohol, may also be used as the serum-free medium. In addition, a commercially available serum-free medium, for example, CD OptiCHO^{™} medium, CHO-S-SFM II medium, or CD CHO medium (Thermo Fisher Scientific, Inc.), IS cho-V^{™} medium (FUJIFILM Irvine Scientific, Inc.), EX-CELL^{™} 302 medium, EX-CELL^{™} Advanced medium, or EX-CELL^{™} 325-PF medium (SAFC Biosciences, Inc.), and the like can also be used as basic media.

To the medium for HSA-hGH fusion protein production, 1 to 50 g/L (for example, 1 to 5 g/L) of a hydrolysate derived from a plant such as soybean, wheat, or rice can be added as appropriate. A most generally used hydrolysate is a soybean-derived protein hydrolysate. However, the HSA-hGH fusion protein can be produced without adding a hydrolysate derived from a plant such as rice, for example, a soybean-derived protein hydrolysate, to the medium for HSA-hGH fusion protein production.

The culture fluid after completion of productive culture is subjected to a chromatography step for purifying the HSA-hGH fusion protein. However, what is subjected to the chromatography step is a culture supernatant obtained by removing cells and the like from the culture fluid. Examples of a method of obtaining a culture supernatant from the culture fluid include filtration using a membrane filter and centrifugal separation.

According to the present invention, each of chromatography steps for purifying the HSA-hGH fusion protein may be carried out in the presence of a nonionic surfactant in order to prevent non-specific adsorption of proteins as necessary. There is no particular limitation on which nonionic surfactant should be used; however, a polysorbate-based surfactant is preferably used, and more preferred is polysorbate 80 or polysorbate 20. The concentration of such a nonionic surfactant is preferably 0.005% (w/v) to 0.1% (w/v), more preferably 0.005% (w/v) to 0.05% (w/v), and for example, 0.01% (w/v) or 0.05% (w/v).

A purification step for the HSA-hGH fusion protein can be carried out at room temperature or a low temperature; however, the purification step can be carried out preferably at a low temperature, particularly at 1°C to 10°C.

According to an embodiment of the present invention, the purification step for the HSA-hGH fusion protein includes a column chromatography step of using a material to which an antibody having an affinity for the HSA-hGH fusion protein is linked, as a stationary phase; a column chromatography step of using a material having an affinity for phosphate group as a stationary phase; a cation exchange column chromatography step; and a size exclusion column chromatography step. However, one or a plurality of chromatography steps can also be added to these chromatography steps. Examples of such additional chromatography steps include a column chromatography step of using a material to which an antibody having an affinity for the HSA-hGH fusion protein is linked, as a stationary phase; a column chromatography step of using a material having an affinity for phosphate group as a stationary phase; a cation exchange column chromatography step; an anion exchange column chromatography step; a hydrophobic column chromatography step; a dye affinity column chromatography step; and a size exclusion column chromatography step.

According to an embodiment of the present invention, the purification step for the HSA-hGH fusion protein includes a column chromatography step of using a material to which an antibody having an affinity for the HSA-hGH fusion protein is linked, as a stationary phase; a column chromatography step of using a material having an affinity for phosphate group as a stationary phase; a cation exchange column chromatography step; and a size exclusion column chromatography step, in this order. However, one or a plurality of chromatography steps can also be added to these chromatography steps. Examples of such additional chromatography steps include a column chromatography step of using a material to which an antibody having an affinity for the HSA-hGH fusion protein is linked, as a stationary phase; a column chromatography step of using a material having an affinity for phosphate group as a stationary phase; a cation exchange column chromatography step; an anion exchange column chromatography step; a hydrophobic column chromatography step; a dye-ligand column chromatography step; and a size exclusion column chromatography step. The additional chromatography may be added between any adjacent steps or may be added as a first chromatography step or a final chromatography step.

The purification step according to an embodiment of the present invention, including a column chromatography step of using a material to which an antibody having an affinity for the HSA-hGH fusion protein is linked, as a stationary phase; a column chromatography step of using a material having an affinity for phosphate group as a stationary phase; a cation exchange column chromatography step; and a size exclusion column chromatography step in this order will be described in detail below.

A first column chromatography step is to use column chromatography that uses a material to which an antibody having an affinity for the HSA-hGH fusion protein is linked, as a stationary phase. Here, the antibody may have an affinity for either HSA or hGH; however, an antibody having an affinity for hGH is preferred. For example, Capture select Human Growth Hormone Affinity Matrix (Thermo Fisher Scientific, Inc.), which includes a carrier to which an antibody to human growth hormone is linked, can be suitably used.

When column chromatography that uses a material to which an antibody having an affinity for hGH is linked, is used as a stationary phase, the HSA-hGH fusion protein is subjected to a column that has been equilibrated in advance with a buffer solution. Here, the type of the buffer solution is not particularly limited; however, a Tris-HCl buffer solution is preferred, and the concentration thereof is preferably 5 to 50 mM, and more preferably 10 to 30 mM. Furthermore, the pH is preferably adjusted to 6.7 to 7.3, more preferably to 6.9 to 7.1, and even more preferably to about 7.0. Elution of the HSA-hGH fusion protein from the column is preferably carried out using glycine-hydrochloric acid. The concentration of glycine-hydrochloric acid is preferably 20 to 100 mM, more preferably 40 to 60 mM, and even more preferably about 50 mM. Furthermore, the pH is preferably adjusted to 2.0 to 4.0, more preferably to 2.8 to 3.2, and even more preferably to about 3.0. The pH of the eluate is immediately adjusted to near neutrality, for example, pH 6.4 to 7.0.

A second column chromatography step is to use a material having an affinity for phosphate group as a stationary phase. As suitable examples of column chromatography using a material having an affinity for phosphate group as a stationary phase, hydroxyapatite column chromatography and fluorapatite column chromatography may be mentioned; however, hydroxyapatite column chromatography is particularly preferred. These are intended to remove contaminants by utilizing an interaction of both metal affinity by calcium ion and cation exchange by phosphate group.

The case of using hydroxyapatite column chromatography will be described in detail below. The carrier to be used for hydroxyapatite chromatography is not particularly limited and may be ceramic or crystalline; however, one of particularly preferred carriers may be CHT Type II, 40 µm (Bio-Rad Laboratories, Inc.).

With regard to the eluate obtained in the first column chromatography step, the pH and the conductivity are adjusted before the eluate is subjected to a hydroxyapatite chromatography column. At this time, the pH is preferably adjusted to 6.5 to 7.5, more preferably to 6.8 to 7.2, and even more preferably to 6.9 to 7.1. Furthermore, the conductivity is preferably adjusted to 0.4 to 0.8 S/m, and more preferably to 0.5 to 0.7 S/m.

The eluate whose pH and conductivity have been adjusted is subjected to the hydroxyapatite chromatography column that has been equilibrated in advance with a buffer solution. Here, the type of the buffer solution is not particularly limited; however, an MES buffer solution is preferred, while the concentration thereof is preferably 5 to 50 mM, more preferably 10 to 30 mM, and for example, 20 mM. Furthermore, the pH of the buffer solution is preferably adjusted to 6.7 to 7.3, more preferably to 6.9 to 7.1, and even more preferably to about 7.0. Furthermore, the buffer solution includes phosphate ions, and the concentration thereof is preferably 0 to 3 mM, more preferably 0 to 2 mM, and for example, 1 mM.

Elution of the HSA-hGH fusion protein from the hydroxyapatite chromatography column is carried out by using a buffer solution having an increased concentration of phosphate ion. The concentration of phosphate at this time is preferably 20 to 50 mM, more preferably 25 to 40 mM, even more preferably 25 to 35 mM, and for example, 30 mM.

A third column chromatography step is to use cation exchange column chromatography and is intended to remove contaminant proteins. There is no particular limitation on which cation exchange resin should be used for the cation exchange column chromatography; however, a weak cation exchange resin is preferred, and a weak cation exchange resin having selectivity based on both hydrophobic interaction and hydrogen bonding formation is more preferred. For example, a weak cation exchange resin having a phenyl group, an amide bond, and a carboxyl group and having selectivity based on hydrophobic interaction and hydrogen bonding formation, such as Capto MMC (GE Healthcare), can be suitably used.

With regard to the eluate obtained in the second column chromatography step, the pH and the conductivity are adjusted before the eluate is subjected to cation exchange column chromatography. At this time, the pH is preferably adjusted to 5.3 to 6.2, more preferably to 5.4 to 6.1, and even more preferably to 5.6 to 5.8. Furthermore, the conductivity is preferably adjusted to 0.5 to 0.9 S/m, and more preferably to 0.6 to 0.8 S/m.

The eluate whose pH and conductivity have been adjusted is subjected to cation exchange column chromatography that has been equilibrated in advance with a buffer solution. Here, the type of the buffer solution is not particularly limited; however, an MES buffer solution is preferable, and the concentration thereof is preferably 30 to 70 mM, more preferably 40 to 60 mM, and for example, 50 mM. Furthermore, the pH of the buffer solution is preferably adjusted to 5.4 to 6.0, more preferably to 5.6 to 5.8, and for example, to 5.7. Furthermore, the buffer solution includes a salt, and when the salt is a neutral salt, the concentration in the buffer solution is preferably 30 to 150 mM, more preferably 50 to 120 mM, even more preferably 80 to 120 mM, and for example, 100 mM. The neutral salt at this time is preferably sodium chloride or potassium chloride; however, the salt is particularly preferably sodium chloride.

Elution of the HSA-hGH fusion protein from cation exchange column chromatography is carried out by using a buffer solution having an increased concentration of a neutral salt. The concentration of the neutral salt at this time is preferably 400 to 600 mM, more preferably 500 to 600 mM, even more preferably 530 to 570 mM, and for example, 550 mM.

A fourth column chromatography step is to use size exclusion chromatography. Size exclusion chromatography is intended to remove, in particular, low molecular weight contaminants such as endotoxin, and oligomers and degradation products of the HSA-hGH fusion protein, based on the molecular size.

In the size exclusion chromatography step, the column is equilibrated in advance. Here, the type of the buffer solution is not particularly limited; however, a phosphate buffer solution is preferred, and the concentration thereof is preferably 5 to 20 mM, more preferably 8 to 12 mM, and for example, 10 mM. The pH of the buffer solution is preferably adjusted to 6.6 to 7.4, more preferably to 7.0 to 7.4, and for example, to 7.2. Furthermore, the buffer solution may contain a disaccharide that can be used as a pharmaceutical additive. Such a disaccharide is preferably sucrose, and the concentration thereof is preferably 60 to 90 mg/mL, more preferably 70 to 80 mg/mL, and for example, 75 mg/mL. Through these first to fourth column chromatography steps, substantially pure HSA-hGH fusion protein is obtained.

In the purification step for the HSA-hGH fusion protein, a virus inactivation step may be added as desired. The virus inactivation step may be carried out between any two chromatography steps or may be carried out before the first chromatography step or after the final chromatography step. Furthermore, the virus inactivation step may be carried out once, may be carried out twice, or may be carried out three or more times, during the purification step for the HSA-hGH fusion protein.

When the chromatography steps include a column chromatography step (first column chromatography step) of using a material to which an antibody having an affinity for the HSA-hGH fusion protein is linked, as a stationary phase; a column chromatography step (second column chromatography step) of using a material having an affinity for phosphate group as a stationary phase; a cation exchange column chromatography step (third column chromatography step); and a size exclusion column chromatography step (fourth column chromatography step) in this order, the virus inactivation step is preferably carried out between the first column chromatography step and the second column chromatography step, or/and after the fourth column chromatography step. There is no particular limitation on which virus inactivation step should be applied; however, a solvent-detergent method or a filter filtration method can be suitably applied. The solvent-detergent method is a method of inactivating virus by adding an organic solvent and a surfactant to a solution in which viruses should be inactivated. When the solvent-detergent method is applied, an organic solvent and a nonionic surfactant are added and mixed into a solution containing the HSA-hGH fusion protein, and this mixed liquid is incubated for, for example, more than 3 hours. The solution to be used for the solvent-surfactant method is not particularly limited as long as the HSA-hGH fusion protein is stably retained for at least two hours; however, a mixture obtained by mixing an organic solvent and a nonsurfactant into a glycine buffer solution, a phosphate buffer solution, an MES buffer solution, a Tris-HCl buffer solution, or a mixture of these, in all of which the pH has been adjusted to near neutrality, can be suitably used. Furthermore, there is also no particular limitation on which nonionic surfactant should be used; however, for example, polysorbate 20, polysorbate 80, and triton X-100 can be used singly or in combination of any of these. Polysorbate 80 is one of particularly suitable nonionic surfactants and can be used singly or in combination with other nonionic surfactants. Furthermore, there is also no particular limitation on which organic solvent should be used; however, for example, tri(n-butyl phosphate) can be used. When polysorbate 80 is used in combination with tri(n-butyl phosphate), the concentration of polysorbate 80 in the mixed liquid is 0.3% to 2%, for example, 1%, the concentration of tri(n-butyl phosphate) is 0.1% to 0.5%, for example, 0.3%, and the mixed liquid is incubated for 2 to 5 hours, for example, for 3 hours.

The filter filtration method is a method of removing viruses by filtering a solution from which viruses should be removed, through a filtration membrane having the performance of removing viruses. The filtration membrane used for the filter filtration method is preferably such that the average pore size is 17 to 21 nm, and the material thereof is, for example, regenerated cellulose. When the filter filtration method is applied, a solution containing the HSA-hGH fusion protein is passed through a virusremoving membrane. For example, virus removal can be more reliably carried out by performing virus inactivation by a solvent-detergent method between the first column chromatography step and the second column chromatography step and performing virus inactivation by a filter filtration method after the fourth column chromatography step.

Incidentally, the method of using a virusremoving membrane can be considered as a virus removal step; however, according to the present invention, the method can also be considered as one method for the virus inactivation step.

According to the present invention, the HSA-hGH fusion protein has increased stability in blood and a prolonged half-life, as compared with the original growth hormone to which HSA is not linked. Although the half-life in blood may vary depending on the route of administration and the dosage, when the HSA-hGH fusion protein is administered to cynomolgus monkeys by subcutaneous injection, the half-life in blood (t_{1/2}β) is, for example, 5 hours or longer, and the fusion protein becomes extremely stable in blood. For example, regarding the half-life in blood (t_{1/2}β) of the HSA mutant-human growth hormone fusion protein, when the fusion protein is subcutaneously administered once to male cynomolgus monkeys at a dose of 0.5 to 10 mg/kg, the half-life in blood (t_{1/2}β) is 5 to 40 hours.

According to the present invention, a medicine containing the HSA mutant-growth hormone fusion protein as an active ingredient can be intravenously, intramuscularly, intraperitoneally, or subcutaneously administered as an injectable preparation.

Human growth hormone mainly has two kinds with different molecular weights, namely, a hormone having a molecular weight of 22 kDa (22 kDa human growth hormone, 22K human growth hormone in the present specification) and a hormone having a molecular weight of 20 kDa (20 kDa human growth hormone, 20K human growth hormone in the present specification). The 22K growth hormone is a protein having an amino acid sequence set forth in SEQ ID NO:9 and composed of 191 amino acids. Usually, when the term "human growth hormone (or hGH)" is said, it means this 22K growth hormone; however, when the term "human growth hormone (or hGH)" is simply said in the present specification, it includes both the 22K human growth hormone and the 20K human growth hormone.

According to the present specification, when the term "22K human growth hormone (or 22KhGH)" is simply said, in addition to the wild-type 22KhGH having an amino acid sequence set forth in SEQ ID NO:9, a 22KhGH mutant in which one amino acid or a plurality of amino acids have been substituted, deleted, and/or added with respect to this wild-type 22KhGH, and which has growth promoting activity, is also included. The number of amino acids that may be substituted, deleted, and/or added is preferably 1 to 8, more preferably 1 to 4, and even more preferably 1 to 2, for each mutation type. Here, the amino acid sequence of the 22KhGH mutant preferably has an identity of 85% or higher, more preferably an identity of 90% or higher, and even more preferably an identity of 95% or higher, with the amino acid sequence of the wild-type 22KhGH set forth in SEQ ID NO:9.

The wild-type 20K human growth hormone corresponds to a protein in which fifteen amino acids from the 32^{nd} to the 46^{th} positions as counted from the N-terminus among the 191 amino acids constituting the wild-type 22K growth hormone (SEQ ID NO:9) have been deleted, the protein having an amino acid sequence composed of 176 amino acids (SEQ ID NO:10) and having growth promoting activity. However, according to the present specification, when the term "20K human growth hormone (or 20KhGH)" is simply said, in addition to the wild-type 20KhGH set forth in SEQ ID NO:10, a mutant of 20KhGH corresponding to a sequence obtained by substituting, deleting, and/or adding one amino acid or a plurality of amino acids with respect to the sequence of the wild-type 20KhGH, the mutant having growth promoting activity, is also included. The number of amino acids that may be substituted, deleted, and/or added is preferably 1 to 8, more preferably 1 to 4, and even more preferably 1 or 2, for each mutation type. Here, the amino acid sequence of the 20KhGH preferably has an identity of 85% or higher, more preferably an identity of 90% or higher, and even more preferably an identity of 95% or higher, with the amino acid sequence of the wild-type 20KhGH set forth in SEQ ID NO:10.

According to the present invention, the position of each mutation and the form thereof (deletion, substitution, or addition) in each of various hGH mutants as compared with normal wild-type hGH can be easily checked by checking the alignment of the amino acid sequences of the two hGHs. According to the present invention, the identity between the amino acid sequence of wild-type hGH and the amino acid sequence of hGH to which mutation has been applied, can be easily calculated by using well-known homology calculation algorithms. Examples of such algorithms include BLAST (Altschul SF, J Mol. Biol., 215, 403-10 (1990)), the similarity search method of Pearson and Lipman (Proc. Natl. Acad. Sci., USA, 85, 2444 (1988)), and the local homology algorithm of Smith and Waterman (Adv. Appl. Math., 2, 482-9 (1981)).

With regard to the substitution of an amino acid in the above-described amino acid sequence of hGH with another amino acid, for example, amino acids classified as the same groups, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxy group (Ser, Thr), and amino acids having a small side chain (Gly, Ala, Ser, Thr, Met), may be mentioned. Substitution with such a similar amino acid is not expected to bring significant changes in the functions of hGH (that is, conservative amino acid substitution).

Preparations containing, as an active ingredient, a hGH having a molecular weight of about 22 kD produced as a recombinant protein by using hGH gene-introduced Escherichia coli (hGH preparations) have been clinically widely used as therapeutic agents for growth hormone deficiency dwarfism, short stature associated with Turner syndrome, Small-for-Gestational Age (SGA) dwarfism, dwarfism associated with Noonan syndrome, dwarfism caused by chronic renal failure, dwarfism associated with Prader-Willi syndrome, and dwarfism associated with cartilage dystrophy. hGH preparations are particularly effective in a case where these diseases are not accompanied by epiphyseal closure. When hGH preparations are administered subcutaneously or intramuscularly, hGH circulates in the blood and exhibits an effect of promoting the growth of a patient by the growth promoting activity of hGH. Furthermore, hGH preparations are widely clinically used as a therapeutic agent for adult growth hormone deficiency. In patients with adult growth hormone deficiency, abnormal lipid metabolism is observed; however, when an hGH preparation is administered, the patient's lipid metabolism is normalized, and the QOL of the patient is improved. Growth hormone is clinically applied also as a therapeutic agent for depletion caused by AIDS. As an hGH preparation for growth hormone deficiency dwarfism, adult growth hormone deficiency, and the like, for example, GROWJECT (registered trademark) is available.

When a fusion protein of an HSA mutant and hGH is produced, regarding a specific method of linking a polypeptide including the amino acid sequence of the HSA mutant and a polypeptide including the amino acid sequence of hGH, for example, a method of producing an expression vector having a DNA fragment incorporated therein, the DNA fragment being a DNA fragment in which a gene encoding one of the polypeptides is linked in-frame downstream of a gene encoding the other polypeptide, and culturing host cells that have been transformed by using this expression vector to express the fusion protein as a recombinant protein, is general and can be utilized for the present invention.

When a fusion protein of an HSA mutant and hGH is produced by a method of expressing the fusion protein in transformed cells as a recombinant protein, a polypeptide including the amino acid sequence of hGH is linked to either the N-terminal side or the C-terminal side of a polypeptide including the amino acid sequence of the HSA mutant directly or indirectly through a linker.

When the polypeptide including the amino acid sequence of hGH is linked to the N-terminal side of the polypeptide including the amino acid sequence of the HSA mutant, an expression vector having a DNA fragment incorporated therein, the DNA fragment being a DNA fragment in which a gene encoding the polypeptide that includes the amino acid sequence of the HSA mutant is linked in-frame downstream of a gene encoding the polypeptide that includes the amino acid sequence of hGH, is used. When the two polypeptides are indirectly linked through a peptide linker, a DNA sequence encoding the linker is disposed in-frame between the genes encoding the two polypeptides.

When the polypeptide including the amino acid sequence of hGH is linked to the C-terminal side of the polypeptide including the amino acid sequence of the HSA mutant, an expression vector having a DNA fragment incorporated therein, the DNA fragment being a DNA fragment in which a gene encoding the polypeptide that includes the amino acid sequence of the HSA mutant is linked in-frame upstream of a gene encoding the polypeptide that includes the amino acid sequence of hGH, is used. When the two polypeptides are indirectly linked through a peptide linker, a DNA sequence encoding the linker is disposed in-frame between the genes encoding the two polypeptides.

According to the present invention, as a suitable example of the fusion protein of an HSA mutant and hGH (one kind of HSA-hGH fusion proteins), an HSA-hGH fusion protein having an amino acid sequence set forth in SEQ ID NO:11, in which the C-terminus of 22K human growth hormone having the amino acid sequence set forth in SEQ ID NO:9 is linked to the N-terminus of HSA (A320T) having the amino acid sequence set forth in SEQ ID NO:3 by peptide bonding, with a linker interposed therebetween, may be mentioned. According to the present invention, a product obtained by linking HSA (A320T) and 22KhGH in this order is referred to as "22K human growth hormone-mHSA" or "22KhGH-mHSA". Similarly, a product in which the N-terminus of 22K human growth hormone is linked to the C-terminus of HSA (A320T) by peptide bonding, with a linker interposed therebetween, is referred to as "mHSA-22K human growth hormone" or "mHSA-22KhGH".

Furthermore, an HSA-hGH fusion protein having an amino acid sequence set forth in SEQ ID NO:12, in which the C-terminus of 20K human growth hormone having the amino acid sequence set forth in SEQ ID NO:10 is linked to the N-terminus of human serum albumin (A320T) having the amino acid sequence set forth in SEQ ID NO:3 by peptide bonding, with a linker interposed therebetween, is referred to as "20K human growth hormone-mHSA" or "20KhGH-mHSA". Similarly, a product in which the N-terminus of 20K human growth hormone is linked to the C-terminus of human serum albumin (A320T) by peptide bonding, with a linker interposed therebetween, is referred to as "mHSA-20K human growth hormone" or "mHSA-22KhGH".

According to the present invention, the HSA-hGH fusion protein has a feature that when the HSA-hGH fusion protein is administered to cynomolgus monkeys by subcutaneous injection, the half-life in blood (t_{1/2}β) is approximately 5 hours or longer, and the fusion protein becomes extremely stable in blood. Although the half-life in blood may vary depending on the dosage, for example, when the fusion proteins are subcutaneously administered once to male cynomolgus monkeys at a dose of 4 mg/kg, the half-life in blood (t_{1/2}β) of mHSA-22KhGH and 22KhGH-mHSA is 20 to 35 hours.

The HSA-hGH fusion protein according to the present invention can be used as a medicine. The HSA-hGH fusion protein can coordinate the functions of human growth hormone and HSA in vivo.

The HSA-hGH fusion protein according to the present invention is extremely stable in blood. Therefore, according to the present invention, human growth hormone can be stabilized in blood to stay in blood in a state of retaining activity over a long period of time. Therefore, when this fusion protein is used as a medicine, the frequency of administration or/and the dosage can be reduced as compared to human growth hormone. For example, human growth hormone needs to be administered daily; however, with this fusion protein, the frequency of administration can be adjusted to, for example, every 3 to 30 days. Furthermore, it is also possible to reduce the total dosage of the medicine during the treatment period to, for example, 1/3 or less (for example, 1/3 to 1/10) in terms of molar ratio.

The HSA-hGH fusion protein according to the present invention can be used as a medicine for growth hormone deficiency dwarfism, short stature associated with Turner syndrome, dwarfism caused by chronic renal failure, dwarfism associated with Prader-Willi syndrome, and dwarfism associated with cartilage dystrophy, SGA dwarfism, and dwarfism associated with Noonan syndrome, as target diseases. hGH preparations are particularly effective in a case where these diseases are not accompanied by epiphyseal closure. In addition to that, the HSA-hGH fusion protein according to the present invention can be used as a medicine for adult growth hormone deficiency, depletion caused by AIDS, and depletion caused by anorexia, as target diseases; however, these diseases are not limited to these, the HSA-hGH fusion protein can be used as a therapeutic agent for diseases in which symptoms can be improved by causing growth promoting activity such as cartilage formation promotion and protein assimilation promotion, and physiological activity such as body composition and lipid metabolism improving action, to be effected for a long time period.

There is no particular limitation on the dosage regimen at the time of administering mHSA-22KhGH to a human for therapeutic purposes, as long as the efficacy of hGH is exhibited. The dosage regimen of mHSA-22KhGH will be described with examples below; however, the dosage regime is not limited to these.

When mHSA-22KhGH is administered to a patient with growth hormone deficiency dwarfism that is not accompanied by epiphyseal closure, a preferred dosage per dose is 0.01 to 0.7 mg/Kg of body weight. When mHSA-22KhGH is administered to a patient with dwarfism associated with Turner syndrome that is not accompanied by epiphyseal closure, a preferred dosage per dose is 0.015 to 1.4 mg/Kg of body weight. When mHSA-22KhGH is administered to a patient with dwarfism caused by chronic renal failure that is not accompanied by epiphyseal closure, a preferred dosage per dose is 0.01 to 1.4 mg/Kg of body weight. When mHSA-22KhGH is administered to a patient with dwarfism associated with Prader-Willi syndrome that is not accompanied by epiphyseal closure, a preferred dosage per dose is 0.012 to 0.98 mg/Kg of body weight. When mHSA-22KhGH is administered to a patient with dwarfism associated with cartilage dystrophy that is not accompanied by epiphyseal closure, a preferred dosage per dose is 0.015 to 1.4 mg/Kg of body weight. When mHSA-22KhGH is administered to a patient with SGA dwarfism that is not accompanied by epiphyseal closure, a preferred dosage per dose is 0.012 to 1.9 mg/Kg of body weight. When mHSA-22KhGH is administered to a patient with adult growth hormone deficiency, a preferred dosage per dose is 0.001 to 0.34 mg/Kg of body weight. When mHSA-22KhGH is administered to a patient with dwarfism associated with Noonan syndrome that is not accompanied by epiphyseal closure, a preferred dosage per dose is 0.013 to 1.8 mg/Kg of body weight. When mHSA-22KhGH is administered to a patient depleted by AIDS, a preferred dosage per dose is 0.005 to 0.4 mg/Kg of body weight. However, the dosage should be appropriately changed depending on the patient's laboratory findings and the like. Furthermore, a preferred administration interval of mHSA-22KhGH for these diseases is once every 7 to 30 days, and the administration interval should be appropriately changed to once every 7 to 14 days, every 10 to 20 days, or every 14 to 21 days, depending on the patient's laboratory findings and the like. Furthermore, the method of administration is preferably subcutaneous injection, intramuscular injection, or intravenous injection, and more preferably subcutaneous injection or intramuscular injection.

A medicine containing the HSA-hGH fusion protein of the present invention as an active ingredient can be administered intravenously, intramuscularly, intraperitoneally, subcutaneously, or intraventricularly as an injectable preparation. Those injectable preparations can be supplied as freeze-dried preparations or aqueous liquid preparations. When the medicine is prepared into an aqueous liquid preparation, the aqueous liquid preparation may be in the form of being filled in a vial or may be supplied as a prefilled-type preparation, which has been filled in advance in a syringe. In the case of a lyophilized preparation, the preparation is dissolved in an aqueous medium to be restored before use and then used.

### EXAMPLES

Hereinafter, the present invention will be described in more detail with reference to Examples; however, the present invention is not intended to be limited to the Examples.

### [Example 1] Construction of vector for 22KhGH-HSA expression

A protein having an amino acid sequence set forth in SEQ ID NO:15, in which the C-terminus of 22KhGH is linked to the N-terminal of wild-type HSA (SEQ ID NO:1), was designated as 22KhGH-HSA. In the amino acid sequence set forth in SEQ ID NO:15, the 1^{st} to 191^{st} amino acid residues correspond to the amino acid sequence of 22KhGH, and the 192^{nd} to 776^{th} amino acid residues correspond to the amino acid sequence of HSA. A DNA having a base sequence set forth in SEQ ID NO:16, which included a gene encoding 22KhGH-HSA (22KhGH-HSA gene), was chemically synthesized. With regard to this sequence, bases 11 to 88 encode a leader peptide of hGH, bases 89 to 661 encode 22KhGH, and bases 662 to 2416 encode HSA. This DNA was digested with restriction enzymes (MluI and NotI) and was incorporated between the MluI site and the NotI site of pE-mIRES-GS-puro, and thereby pE-mIRES-GS-puro (22KhGH-HSA), which is a vector for 22KhGH-HSA expression, was constructed. Incidentally, regarding pE-mIRES-GS-puro, the production method is described in WO 2012/063799 and the like and is well known.

### [Example 2] Construction of vector for 22KhGH-mHSA expression

A protein having an amino acid sequence set forth in SEQ ID NO:11, in which the C-terminus of 22KhGH (SEQ ID NO:9) was linked to the N-terminus of HSA (A320T) (SEQ ID NO:3), was designated as 22KhGH-mHSA. A DNA fragment including a gene encoding 22KhGH-mHSA was amplified by PCR by using the pE-mIRES-GS-puro (22KhGH-HSA) produced in Example 1 as a template and using primer YA082 (SEQ ID NO:13) and primer YA083 (SEQ ID NO:14). This DNA fragment was self-annealed to construct pE-mIRES-GS-puro (22KhGH-mHSA), which was a vector for 22KhGH-mHSA expression.

### [Example 3] Production of 22KhGH-mHSA expressing cell line

Cells for expressing 22KhGH-mHSA were produced as follows. pE-mIRES-GS-puro (22KhGH-mHSA), which was a vector for 22KhGH-mHSA expression, produced in Example 2 was introduced into CHO-K1 cells, which are cells derived from the ovary of a Chinese hamster, by using a Gene Pulser Xcell electroporation system (Bio-Rad Laboratories, Inc.). Cells having the expression vector introduced therein were selectively cultured by using CD OptiCHO^{™} medium (Thermo Fisher Scientific, Inc.) including 16 µM thymidine, 100 µM hypoxanthine, 10 mg/L insulin, L-methionine sulfoximine (Sigma-Aldrich Corp.), and puromycin (Sigma-Aldrich Corp.). During the selective culture, the concentrations of L-methionine sulfoximine and puromycin were increased stepwise to finally set the concentration of L-methionine sulfoximine to 300 µM and the concentration of puromycin to 10 µg/mL, and cells exhibiting higher drug resistance were selectively proliferated. Next, the cells selected by selective culture were seeded onto a 96-well plate such that not more than one cell was seeded per well by a limiting dilution method, the cells were cultured for about 10 days until single cell-derived colonies were formed to clone the cells. The cloned cells were further cultured to proliferate and then were suspended in CD OptiCHO^{™} medium containing 16 µM thymidine, 100 µM hypoxanthine, 300 µM L-methionine sulfoximine, and 10% (v/v) DMSO, and subsequently the cells were dispensed in cryotubes and stored in liquid nitrogen. These cells were designated as 22KhGH-mHSA expressing cell line.

### [Example 4] Preculture of 22KhGH-mHSA expressing cells

The 22KhGH-mHSA expressing cell line produced in Example 3 was thawed in a water bath at 37°C, suspended in EX-CELL Advanced medium (medium for preculture, Sigma-Aldrich Corp.) of a serum-free medium including 16 µM thymidine, 100 µM hypoxanthine, and 300 µM L-methionine sulfoximine, and centrifuged to precipitate the cells, and the supernatant was removed. The precipitated cells were suspended in the medium for preculture at a density of 2×10⁵ cells/mL or more, and the cells were cultured for 2 to 4 days at 37°C in the presence of 5% CO₂. This culture was repeated while expanding the culture scale until the number of cells increased to at least 1.0×10¹¹ cells.

### [Example 5] Productive culture of 22KhGH-mHSA expressing cells

The cells proliferated in preculture were suspended in EX-CELL Advanced medium (medium for productive culture, Sigma-Aldrich Corp.), which was a serum-free medium including 16 µM thymidine and 100 µM hypoxanthine, in a volume of 200 L at a cell concentration of a density of 4×10⁵ cells/mL. This suspension was cultured in a single-use culture tank of an Xcellerex 200L culture system (XDR200) for 10 days while being stirred with an impeller at a rate of 100 rpm, with the culture system maintained at 37°C, a dissolved oxygen content of 40%, and a pH of 6.9.

### [Example 6] Purification of 22KhGH-mHSA

### (Purification step 1: Harvest step/concentration 1 step)

After completion of the productive culture, the culture fluid was filtered by using Millistak+ (registered trademark) HC Pod Filter Grade D0HC (1.1 m² × 2, Merck KGaA) and then was filtered by using Millistak+ HC Pod Filter Grade X0HC (1.1 m² × 1, Merck KGaA) to remove cells, and the resultant was further filtered through Opticap SHC XL3 (pore size: 0.5/0.2 µm, Merck KGaA) to obtain a culture supernatant. The obtained culture supernatant was concentrated by using an ultrafiltration membrane device (equipped with Pellicon 3 Cassette Ultracel PLCTK membrane, 1.14 m², Merck KGaA) which was washed in advance with pure water and equilibrated with PBS and having a molecular weight cutoff of 30 kDa. The solution after concentration was collected from the ultrafiltration membrane device. Furthermore, the inside of the device was washed by passing PBS therethrough, this washing liquid was combined with the solution after concentration that had been collected first, subsequently the weight was adjusted to about 15 kg, and this was used as concentrated culture fluid. Next, the concentrated culture fluid was filtered by using a hydrophilic filter (Opticap SHC XL600, Merck KGaA) having a maximum pore size of 0.5 µm and a minimum pore size of 0.2 µm. After the filtration, it was confirmed that the pH and conductivity of the concentrated culture fluid were 7.0 ± 0.3 and 1.2 ± 0.4 S/m, respectively.

### (Purification step 2: First chromatography step (affinity column chromatography step))

A QS column (column volume: 4.2 to 5.2 L, bed height: 15.0 ± 1.5 cm, Merck KGaA) packed with Capture select Human Growth Hormone Affinity Matrix (Thermo Fisher Scientific, Inc.) was washed with a 50 mM glycine hydrochloric acid buffer solution (pH 3.0) in a volume 1 times the volume of the column and a 0.01 M aqueous solution of sodium hydroxide in a volume 1 times the volume of the column and then was equilibrated with a 20 mM Tris-HCl buffer solution (pH 7.0) in a volume 4 times the volume of the column. Next, a 1/4 amount of the concentrated culture fluid obtained in purification step 1 was loaded on the column, and 22KhGH-mHSA was adsorbed to the column. Next, the column was washed with a 20 mM Tris-HCl buffer solution (pH 7.0) containing 200 mM arginine and 0.05% (w/v) polysorbate 80 in a volume 5 times the volume of the column, and a 20 mM Tris-HCl buffer solution (pH 7.0) in a volume 5 times the volume of the column. Next, a 50 mM glycine hydrochloric acid buffer solution (pH 3.0) in a volume 5 times the volume of the column was supplied to the column, and 22KhGH-mHSA was eluted. The eluate was collected in a container containing a 250 mM MES buffer solution (pH 7.0) in a capacity of 1/5 the amount of the eluate in advance and was immediately neutralized such that the pH became 6.7 ± 0.3. Purification step 2 was carried out at a cold temperature of 4°C to 8°C, and the linear flow rate of the solution to be supplied to the column was set to 200 cm/hour throughout the purification step 2. Furthermore, the upper limit of 22KhGH-mHSA to be loaded per 1 L of the affinity column carrier was set to 7 g.

### (Purification step 3: Virus inactivation step)

The eluate obtained in purification step 2 was warmed to 25°C, a 6.0% (v/v) aqueous solution of tri(n-butyl) phosphate containing 20.0% (w/v) polysorbate 80 in a capacity of 1/19 the liquid amount of the eluate was added to the eluate, and the mixture was stirred at 25°C for 3 hours.

### (Purification step 4: Second chromatography step (hydroxyapatite column chromatography step))

The fraction that had been subjected to a virus inactivation treatment in purification step 3 was cooled to 8°C, an appropriate amount of a 20 mM MES buffer solution (pH 7.0) containing a 1 M Tris-HCl buffer solution (pH 8.8) and 4 M NaCl was added thereto to adjust the pH and the conductivity to 7.0 ± 0.1 and 0.6 ± 0.1 S/m, respectively, and the resultant was filtered by using a hydrophilic filter (Merck KGaA) having a pore size of 0.2 µm. Next, the following chromatography was carried out under refrigeration (linear flow rate: 200 cm/hour).

A QS column (column volume: 8.8 to 10.8 L, bed height: 20.0 ± 2.0 cm, Merck KGaA) packed with CHT Type II, 40 µm (Bio-Rad Laboratories, Inc.), which was a hydroxyapatite carrier, was washed with a 200 mM phosphate buffer solution (pH 7.0), subsequently a 1 M aqueous solution of NaOH, and a 200 mM phosphate buffer solution (pH 7.0), and then the column was equilibrated with a 20 mM MES Buffer solution (pH 7.0) containing 50 mM NaCl and 1 mM sodium dihydrogen phosphate in a volume 4 times the volume of the column. Next, the above-described liquid was loaded on the column, and 22KhGH-mHSA was adsorbed to the column. Next, the column was washed with a 20 mM MES buffer solution (pH 7.0) containing 50 mM NaCl and 1 mM sodium dihydrogen phosphate in a volume 3 times the volume of the column. Next, a 20 mM MES buffer solution (pH 7.0) containing 50 mM NaCl and 30 mM sodium dihydrogen phosphate was supplied to the column to elute 22KhGH-mHSA. Purification step 4 was carried out at a cold temperature of 4°C to 8°C, and the linear flow rate of the solution supplied to the column was set to 200 cm/hour throughout the purification step 4. Furthermore, the upper limit of 22KhGH-mHSA to be loaded per 1 L of the hydroxyapatite carrier was set to 13 g.

### (Purification step 5: Third chromatography step (multimodal weak cation exchange column chromatography step))

To the eluate obtained in purification step 4, a 20 mM MES buffer solution (pH 5.7) containing 50 mM NaCl in the same volume as the volume of this eluate was added, subsequently dilute hydrochloric acid was added thereto to adjust the pH and the conductivity to 5.7 ± 0.1 and 0.7 ± 0.1 S/m, respectively. Subsequently, the mixture was filtered by using a hydrophilic filter (Merck KGaA) having a pore size of 0.5/0.2 µm.

A prepacked column RTP Capto MMC 10L (column volume: 8.8 to 10.8 L, bed height: 20.0 ± 2.0 cm, Merck KGaA) was washed by using a 1 M aqueous solution of NaOH and then a 50 mM phosphate buffer solution (pH 7.0) containing 1 M NaCl and then was equilibrated with a 50 mM MES buffer solution (pH 5.7) containing 100 mM NaCl in a volume 4 times the volume of the column. Next, the above-described liquid was loaded on the column, and 22KhGH-mHSA was adsorbed to the column. Next, the column was washed with a 50 mM MES buffer solution (pH 5.7) containing 100 mM NaCl in a volume 5 times the volume of the column. Next, a 50 mM MES buffer solution (pH 5.7) containing 550 mM NaCl was supplied to the column to elute 22KhGH-mHSA. Purification step 5 was carried out at a cold temperature of 4°C to 8°C, and the linear flow rate of the solution to be supplied to the column was set to 200 cm/hour throughout the purification step 5. Furthermore, the upper limit of 22KhGH-mHSA to be loaded per 1 L of the multimodal weak cation exchange carrier was set to 11.5 g.

### (Purification step 6: Concentration 2 step)

Pure water was passed through an ultrafiltration membrane (Pellicon 3 Cassette Ultracel PLCTK membrane 1.14 m², Merck KGaA) having a molecular weight cutoff of 30 kDa to sufficiently wash the membrane, and then the ultrafiltration membrane was equilibrated with a 10 mM phosphate buffer solution (pH 7.2) containing 75 mg/mL sucrose. The eluate obtained in purification step 5 was concentrated by using this ultrafiltration membrane. To the solution after concentration, a 10 mM phosphate buffer solution (pH 7.2) containing 75 mg/mL sucrose was added to adjust the light absorbance (280 nm) to 23 ± 3. This concentration step was carried out at room temperature.

### (Purification step 7: Fourth chromatography step (size exclusion column chromatography step))

The concentrated liquid obtained in purification step 6 was filtered by using a hydrophilic filter (Merck KGaA) having a pore size of 0.5/0.2 µm. A QS column (column volume: 25.4 to 31.1 L, bed height: 40.0 ± 4.0 cm, Merck KGaA) packed with Fractogel^{™} BioSEC resin (Merck KGaA), which is a resin for size exclusion chromatography, was washed with a 0.5 M aqueous solution of NaOH, and then the column was equilibrated with a 10 mM phosphate buffer solution (pH 7.2) containing 75 mg/mL sucrose. Next, the above-described liquid was loaded on the column, and subsequently a 10 mM phosphate buffer solution (pH 7.2) containing 75 mg/mL sucrose was supplied thereto. At this time, an absorptiometer for continuously measuring the absorbance of the eluate was disposed in the flow channel of the eluate from the size exclusion column, the absorbance at 280 nm was monitored, a fraction showing an absorption peak at 280 nm was collected as the fraction including 22KhGH-mHSA, and this was used as a 22KhGH-mHSA purified product. Purification step 7 was carried out at room temperature, and the linear flow rate of the solution to be supplied to the column was set to 30 cm/hour or less throughout the purification step 7. Furthermore, the liquid amount of the filtrate containing 22KhGH-mHSA to be loaded on the multimodal weak cation exchange carrier was set to be 8% or less of the volume of the carrier.

### (Purification step 8: Concentration 3 step)

A hollow fiber membrane (ReadyToProcess Hollow Fiber Cartridge, molecular weight cutoff 30 kDa, GE Healthcare Systems) was equilibrated with a 10 mM phosphate buffer solution (pH 7.2) containing 75 mg/mL sucrose. The 22KhGH-mHSA purified product obtained in purification step 7 was concentrated by using this hollow fiber membrane, and the concentration of 22KhGH-mHSA was adjusted to 85 mg/mL. To the obtained concentrated liquid, a 10 mM phosphate buffer solution (pH 7.2) containing 90 mg/mL Poloxamer 188 and 75 mg/mL sucrose in a capacity of 1/29 of the liquid amount of the concentrated liquid was added and mixed, and the mixture was filtered by using a hydrophilic filter (Merck KGaA) having a pore size of 0.5/0.2 µm. This concentration step was carried out at room temperature.

### (Purification step 9: Virus removal step)

A 10 mM phosphate buffer solution (pH 7.2) containing 3 mg/mL Poloxamer 188 and 75 mg/mL sucrose was produced. A virus removal membrane (Planova 20 N, membrane area: 0.12 m², material: regenerated cellulose, Asahi Kasei Medical Co., Ltd.) was equilibrated by using this buffer solution. The filtrate obtained in purification step 8 was passed through this virus removal membrane at a pressure of 98 kPa or less to be filtered. This virus removal step was carried out at room temperature.

### (Purification step 10: Drug substance conversion step)

The filtrate obtained in purification step 9 was filtered at room temperature through a hydrophilic filter (Merck KGaA) having a pore size of 0.2 µm, and 22KhGH-mHSA drug substance was obtained.

### [Example 7] Quantification of 22KhGH-mHSA in each purification step

The amount of 22KhGH-mHSA after each of the above-described purification steps was quantified by using the method described in the following Example 8. As shown in Table 1, in the above-described purification steps, 111.08 g of 22KhGH-mHSA was subjected to the affinity column chromatography step, which was the first chromatography step, and finally 65.87 g of a purified product of 22KhGH-mHSA was obtained. That is, the collection ratio of 22KhGH-mHSA in the above-described purification method was 59%, and it was found that this purification method is highly efficient as a purification method for 22KhGH-mHSA. Incidentally, in Table 1, the term "collection ratio/step" means the ratio of the amount of collected 22KhGH-mHSA with respect to the amount of loaded 22KhGH-mHSA for each of the chromatography steps (step), and the term "collection ratio/overall" means the ratio of the amount of 22KhGH-mHSA collected in each step with respect to the initial amount of 22KhGH-mHSA provided to the chromatography step.

### [Table 1]

**Table 1 Collection ratio of 22KhGH-mHSA in each purification step**

| Chromatography step | 22KhGH-mHSA | | | |
|---|---|---|---|---|
| | Amount of loaded protein (g) | Amount of collected protein (g) | Collection ratio/step (%) | Collection ratio/overall (%) |
| Affinity column | 111.08 | 122.31 | 110 | 110 |
| Hydroxyapatite column | 100.31 | 103.75 | 103 | 93 |
| Multimodal weak cation exchange column | 103.12 | 76.26 | 74 | 69 |
| Size exclusion column | 78.70 | 65.87 | 84 | 59 |

### [Example 8] Method for quantifying 22KhGH-mHSA according to Bradford method

A purified product of 22KhGH-mHSA produced by the present invention and quantified by an amino acid analysis method was diluted with water so as to obtain a theoretical protein concentration of 1.0 mg/mL, and this was designated as standard solution 1. Incidentally, the amount of the prepared liquid was adjusted to be 800 µL or more. Next, 200 µL of the standard solution 1 and 50 µL of pure water were mixed so as to obtain a theoretical protein concentration of 0.8 mg/mL, and this was designated as standard solution 2. In such order, 120 µL, 150 µL, and 400 µL of water was mixed into 180 µL, 100 µL, and 100 µL of the standard solution 1, respectively, so as to obtain theoretical protein concentrations of 0.6 mg/mL, 0.4 mg/mL, and 0.2 mg/mL, respectively, and the mixtures were designated as standard solution 3, standard solution 4, and standard solution 5, respectively. 50 µL or more of a test substance was collected and diluted with water such that the protein concentration would be in the range of 0.4 to 0.8 mg/mL, and this was used as a sample solution. The standard solutions and the sample solution were prepared at the time of use.

5 mL each of Coomassie reagent (Pierce Coomassie Assay Reagent included in Pierce^{™} Coomassie (Bradford) Protein Assay Kit, Thermo Fisher Scientific, Inc.) was collected in a 15-mL centrifuge tube. 100 µL each of water (for blank), the standard solutions 1 to 5 and the sample solution were added to the Coomassie reagent collected in the centrifuge tubes, and the mixtures were gently inverted and mixed immediately after the addition of each solution. The mixtures were left to stand still at room temperature for 10 minutes to react. After the reaction, the absorbance at 595 nm was quickly measured with a spectrophotometer (UV-2600, Shimadzu Corp.). The above-described reaction operation using Coomassie reagent was carried out within 2 hours after the preparation of each solution. A calibration curve was created from the measured values of the standard solutions, the measured value of each test substance was interpolated into this calibration curve, and thereby the concentration of 22KhGH-mHSA included in each test substance was calculated.

### [Example 9] Evaluation of purity of 22KhGH-mHSA purified product by SE-HPLC analysis

An SE-HPLC analysis was performed by using LC-20A system (system controller, online degassing unit, liquid feeding unit, autosampler, column oven, and ultraviolet-visible detector) manufactured by Shimadzu Corporation. A TSKgel G3000SW_{XL} column (inner diameter 7.8 mm, length 30 cm, TOSOH Corp.) was applied to the LC-20A system, the column was equilibrated by passing a phosphate buffer solution (200 mM sodium phosphate containing 200 mM NaCl) therethrough at a flow rate of 0.7 mL/min, and then 10 µL of a solution containing 22KhGH-mHSA at a concentration of 2.0 mg/mL was loaded on this column. Similarly, a diluted sample solution (10 mM phosphate buffer solution containing 75 mg/mL sucrose and 3 mg/mL Poloxamer) was loaded at the same flow rate, the absorbance at 215 nm was monitored, and thereby an elution profile was created. A purified product of 22KhGH-mHSA obtained by the above-described production method showed only a single peak approximately corresponding to the monomer of 22KhGH-mHSA in the SE-HPLC analysis (Fig. 1).

The results of the above-described evaluation of the purity of 22KhGH-mHSA show that 22KhGH-mHSA purified by the above-described purification step has high purity that allows the protein to be used as it is as a therapeutic agent for growth hormone deficiency dwarfism and the like.

### INDUSTRIAL APPLICABILITY

According to the present invention, for example, a drug substance of a fusion protein of serum albumin and growth hormone, which can be used as a therapeutic agent for growth hormone deficiency dwarfism, can be provided.

### SEQUENCE LISTING FREE TEXT

SEQ ID NO:1: Amino acid sequence of wild-type human serum albumin
SEQ ID NO:2: Amino acid sequence of human serum albumin Redhill
SEQ ID NO:3: Amino acid sequence of human serum albumin mutant (A320T)
SEQ ID NO:4: Example 1 of linker amino acid sequence
SEQ ID NO:5: Example 2 of linker amino acid sequence
SEQ ID NO:6: Example 3 of linker amino acid sequence
SEQ ID NO:7: Partial base sequence of wild-type mouse encephalomyelitis virus-derived internal ribosome entry site
SEQ ID NO:8: Partial base sequence of mutant mouse encephalomyelitis virus-derived internal ribosome entry site, synthesized
SEQ ID NO:9: Amino acid sequence of 22K human growth hormone
SEQ ID NO:10: Amino acid sequence of 20K human growth hormone
SEQ ID NO:11: Amino acid sequence of 22KhGH-mHSA
SEQ ID NO:12: Amino acid sequence of 20KhGH-mHSA
SEQ ID NO:13: Primer YA082, synthetic sequence
SEQ ID NO:14: Primer YA083, synthetic sequence
SEQ ID NO:15: Amino acid sequence of 22KhGH-HSA
SEQ ID NO:16: Base sequence including 22KhGH-HSA gene, synthetic sequence

### SEQUENCE LISTING

1225JP_ST25.TXT

## Claims

1. A method for producing a fusion protein of human serum albumin and human growth hormone,
the method including:
(a) a step of culturing a mammalian cell capable of producing the protein in a serum-free medium and causing the protein to be secreted into the culture fluid;
(b) a step of collecting a culture supernatant by removing the mammalian cell from the culture fluid obtained in the step (a); and
(c) a step of purifying the protein from the culture supernatant obtained in the step (b), by using column chromatography using a material to which an antibody having an affinity for the protein is bound as a stationary phase, column chromatography using a material having an affinity for phosphate group as a stationary phase, cation exchange column chromatography, and size exclusion column chromatography.

2. The production method according to claim 1, wherein in the step (c), column chromatography using a material in which an antibody having an affinity for the protein is bound as a stationary phase, column chromatography using a material having an affinity for phosphate group as a stationary phase, cation exchange column chromatography, and size exclusion column chromatography are used in this order.

3. The production method according to claim 1 or 2, wherein the antibody having an affinity for the protein has an affinity to human serum albumin or human growth hormone.

4. The production method according to claim 1 or 2, wherein the antibody having an affinity for the fusion protein has an affinity for human growth hormone.

5. The production method according to any one of claims 1 to 4, wherein the material having an affinity for the phosphate group is either fluoroapatite or hydroxyapatite.

6. The production method according to any one of claims 1 to 4, wherein the material having an affinity for the phosphate group is hydroxyapatite.

7. The production method according to any one of claims 1 to 6, wherein a cation exchanger used for the cation exchange column chromatography is a weak cation exchanger.

8. The production method according to claim 7, wherein the weak cation exchanger retains selectivity based on both hydrophobic interaction and hydrophobic bond formation.

9. The production method according to any one of claims 1 to 8, further including a step for inactivating viruses.

10. The production method according to claim 9, wherein an eluate of the protein obtained by column chromatography using a material to which an antibody having an affinity for the protein is bound as a stationary phase, is subjected to inactivation of viruses.

11. The production method according to claim 9, wherein an eluate of the protein obtained by the size exclusion column chromatography is subjected to inactivation of viruses.

12. The production method according to any one of claims 1 to 8, wherein the production method includes two times of the step for inactivating viruses.

13. The production method according to claim 12, wherein an eluate of the protein obtained by column chromatography using a material to which an antibody having an affinity for the protein is bound as a stationary phase, and an eluate of the protein obtained by the size exclusion column chromatography are subjected to inactivation of viruses.

14. The production method according to claim 12 or 13, wherein one time of the step for inactivating viruses is carried out by a method of inactivating viruses by adding a nonionic surfactant to a solution containing HSA-hGH fusion protein, and the other one time is carried out by a method of passing the solution containing HSA-hGH fusion protein through a filtration membrane.

15. The production method according to any one of claims 1 to 14, wherein the protein is formed such that human serum albumin is linked by a peptide bond to the C-terminal side of human growth hormone directly or via a linker sequence.

16. The production method according to claim 15, wherein the linker sequence includes an amino acid sequence selected from the group consisting of one glycine, one serine, amino acid sequence (Gly-Ser), amino acid sequence (Gly-Gly-Ser), and an amino acid sequence consisting of one to ten consecutive sequences of these amino acid sequences.

17. The production method according to claim 16, wherein the linker sequence has an amino acid sequence represented by amino acid sequence (Gly-Ser).

18. The production method according to any one of claims 1 to 14, wherein the amino acid sequence of the protein has an identity of 85% or higher with an amino acid sequence set forth in SEQ ID NO:11.

19. The production method according to any one of claims 1 to 14, wherein the amino acid sequence of the protein has an identity of 95% or higher with the amino acid sequence set forth in SEQ ID NO:11.

20. The production method according to any one of claims 1 to 14, wherein the amino acid sequence of the protein has the amino acid sequence set forth in SEQ ID NO:11.


SEQUENCE LISTING

<110>  JCR Pharmaceuticals Co., Ltd.

<120>  Method for Producing Fusion Protein of Human Serum Albumin and Human
Growth Hormone

<130>  1225JP

<150>  P2019-190600
<151>  2019-10-17

<160>  16

<170>  PatentIn version 3.5

<210>  1
<211>  585
<212>  PRT
<213>  Homo sapiens

<400>  1

<210> 2
<211> 586
<212> PRT
<213> Homo sapiens

<400> 2

Arg Asp Ala His Lys Ser Glu Val Ala His Arg Phe Lys Asp Leu Gly
1               5                   10                  15

Glu Glu Asn Phe Lys Ala Leu Val Leu Ile Ala Phe Ala Gln Tyr Leu
            20                  25                  30

Gln Gln Cys Pro Phe Glu Asp His Val Lys Leu Val Asn Glu Val Thr
        35                  40                  45

Glu Phe Ala Lys Thr Cys Val Ala Asp Glu Ser Ala Glu Asn Cys Asp
    50                  55                  60

Lys Ser Leu His Thr Leu Phe Gly Asp Lys Leu Cys Thr Val Ala Thr
65                  70                  75                  80

Leu Arg Glu Thr Tyr Gly Glu Met Ala Asp Cys Cys Ala Lys Gln Glu
                85                  90                  95

Pro Glu Arg Asn Glu Cys Phe Leu Gln His Lys Asp Asp Asn Pro Asn
            100                 105                 110

Leu Pro Arg Leu Val Arg Pro Glu Val Asp Val Met Cys Thr Ala Phe
        115                 120                 125

His Asp Asn Glu Glu Thr Phe Leu Lys Lys Tyr Leu Tyr Glu Ile Ala
    130                 135                 140

Arg Arg His Pro Tyr Phe Tyr Ala Pro Glu Leu Leu Phe Phe Ala Lys
145                 150                 155                 160

Arg Tyr Lys Ala Ala Phe Thr Glu Cys Cys Gln Ala Ala Asp Lys Ala
                165                 170                 175

Ala Cys Leu Leu Pro Lys Leu Asp Glu Leu Arg Asp Glu Gly Lys Ala
            180                 185                 190

Ser Ser Ala Lys Gln Arg Leu Lys Cys Ala Ser Leu Gln Lys Phe Gly
        195                 200                 205

Glu Arg Ala Phe Lys Ala Trp Ala Val Ala Arg Leu Ser Gln Arg Phe
    210                 215                 220

Pro Lys Ala Glu Phe Ala Glu Val Ser Lys Leu Val Thr Asp Leu Thr
225                 230                 235                 240

Lys Val His Thr Glu Cys Cys His Gly Asp Leu Leu Glu Cys Ala Asp
                245                 250                 255

Asp Arg Ala Asp Leu Ala Lys Tyr Ile Cys Glu Asn Gln Asp Ser Ile
            260                 265                 270

Ser Ser Lys Leu Lys Glu Cys Cys Glu Lys Pro Leu Leu Glu Lys Ser
        275                 280                 285

His Cys Ile Ala Glu Val Glu Asn Asp Glu Met Pro Ala Asp Leu Pro
    290                 295                 300

Ser Leu Ala Ala Asp Phe Val Glu Ser Lys Asp Val Cys Lys Asn Tyr
305                 310                 315                 320

Thr Glu Ala Lys Asp Val Phe Leu Gly Met Phe Leu Tyr Glu Tyr Ala
                325                 330                 335

Arg Arg His Pro Asp Tyr Ser Val Val Leu Leu Leu Arg Leu Ala Lys
            340                 345                 350

Thr Tyr Glu Thr Thr Leu Glu Lys Cys Cys Ala Ala Ala Asp Pro His
        355                 360                 365

Glu Cys Tyr Ala Lys Val Phe Asp Glu Phe Lys Pro Leu Val Glu Glu
    370                 375                 380

Pro Gln Asn Leu Ile Lys Gln Asn Cys Glu Leu Phe Glu Gln Leu Gly
385                 390                 395                 400

Glu Tyr Lys Phe Gln Asn Ala Leu Leu Val Arg Tyr Thr Lys Lys Val
                405                 410                 415

Pro Gln Val Ser Thr Pro Thr Leu Val Glu Val Ser Arg Asn Leu Gly
            420                 425                 430

Lys Val Gly Ser Lys Cys Cys Lys His Pro Glu Ala Lys Arg Met Pro
        435                 440                 445

Cys Ala Glu Asp Tyr Leu Ser Val Val Leu Asn Gln Leu Cys Val Leu
    450                 455                 460

His Glu Lys Thr Pro Val Ser Asp Arg Val Thr Lys Cys Cys Thr Glu
465                 470                 475                 480

Ser Leu Val Asn Arg Arg Pro Cys Phe Ser Ala Leu Glu Val Asp Glu
                485                 490                 495

Thr Tyr Val Pro Lys Glu Phe Asn Ala Glu Thr Phe Thr Phe His Ala
            500                 505                 510

Asp Ile Cys Thr Leu Ser Glu Lys Glu Arg Gln Ile Lys Lys Gln Thr
        515                 520                 525

Ala Leu Val Glu Leu Val Lys His Lys Pro Lys Ala Thr Lys Glu Gln
    530                 535                 540

Leu Lys Ala Val Met Asp Asp Phe Ala Ala Phe Val Glu Lys Cys Cys

545                 550                 555                 560

Lys Ala Asp Asp Lys Glu Thr Cys Phe Ala Glu Glu Gly Lys Lys Leu
                565                 570                 575

Val Ala Ala Ser Gln Ala Ala Leu Gly Leu
            580                 585

<210>  3
<211>  585
<212>  PRT
<213>  Artificial Sequence

<220>
<223>  Amino acid sequence of human serum albumin mutant (A320T)

<400>  3

Asp Ala His Lys Ser Glu Val Ala His Arg Phe Lys Asp Leu Gly Glu
1               5                   10                  15

Glu Asn Phe Lys Ala Leu Val Leu Ile Ala Phe Ala Gln Tyr Leu Gln
            20                  25                  30

Gln Cys Pro Phe Glu Asp His Val Lys Leu Val Asn Glu Val Thr Glu
        35                  40                  45

Phe Ala Lys Thr Cys Val Ala Asp Glu Ser Ala Glu Asn Cys Asp Lys
    50                  55                  60

Ser Leu His Thr Leu Phe Gly Asp Lys Leu Cys Thr Val Ala Thr Leu
65                  70                  75                  80

Arg Glu Thr Tyr Gly Glu Met Ala Asp Cys Cys Ala Lys Gln Glu Pro
                85                  90                  95

Glu Arg Asn Glu Cys Phe Leu Gln His Lys Asp Asp Asn Pro Asn Leu
            100                 105                 110

Pro Arg Leu Val Arg Pro Glu Val Asp Val Met Cys Thr Ala Phe His
        115                 120                 125

Asp Asn Glu Glu Thr Phe Leu Lys Lys Tyr Leu Tyr Glu Ile Ala Arg
    130                 135                 140

Arg His Pro Tyr Phe Tyr Ala Pro Glu Leu Leu Phe Phe Ala Lys Arg
145                 150                 155                 160

Tyr Lys Ala Ala Phe Thr Glu Cys Cys Gln Ala Ala Asp Lys Ala Ala
                165                 170                 175

Cys Leu Leu Pro Lys Leu Asp Glu Leu Arg Asp Glu Gly Lys Ala Ser
            180                 185                 190

Ser Ala Lys Gln Arg Leu Lys Cys Ala Ser Leu Gln Lys Phe Gly Glu
        195                 200                 205

Arg Ala Phe Lys Ala Trp Ala Val Ala Arg Leu Ser Gln Arg Phe Pro
    210                 215                 220

Lys Ala Glu Phe Ala Glu Val Ser Lys Leu Val Thr Asp Leu Thr Lys
225                 230                 235                 240

Val His Thr Glu Cys Cys His Gly Asp Leu Leu Glu Cys Ala Asp Asp
                245                 250                 255

Arg Ala Asp Leu Ala Lys Tyr Ile Cys Glu Asn Gln Asp Ser Ile Ser
            260                 265                 270

Ser Lys Leu Lys Glu Cys Cys Glu Lys Pro Leu Leu Glu Lys Ser His
        275                 280                 285

Cys Ile Ala Glu Val Glu Asn Asp Glu Met Pro Ala Asp Leu Pro Ser
    290                 295                 300

Leu Ala Ala Asp Phe Val Glu Ser Lys Asp Val Cys Lys Asn Tyr Thr
305                 310                 315                 320

Glu Ala Lys Asp Val Phe Leu Gly Met Phe Leu Tyr Glu Tyr Ala Arg
                325                 330                 335

Arg His Pro Asp Tyr Ser Val Val Leu Leu Leu Arg Leu Ala Lys Thr
            340                 345                 350

Tyr Glu Thr Thr Leu Glu Lys Cys Cys Ala Ala Ala Asp Pro His Glu
        355                 360                 365

Cys Tyr Ala Lys Val Phe Asp Glu Phe Lys Pro Leu Val Glu Glu Pro
    370                 375                 380

Gln Asn Leu Ile Lys Gln Asn Cys Glu Leu Phe Glu Gln Leu Gly Glu
385                 390                 395                 400

Tyr Lys Phe Gln Asn Ala Leu Leu Val Arg Tyr Thr Lys Lys Val Pro
                405                 410                 415

Gln Val Ser Thr Pro Thr Leu Val Glu Val Ser Arg Asn Leu Gly Lys

420                 425                 430

Val Gly Ser Lys Cys Cys Lys His Pro Glu Ala Lys Arg Met Pro Cys
        435                 440                 445

Ala Glu Asp Tyr Leu Ser Val Val Leu Asn Gln Leu Cys Val Leu His
    450                 455                 460

Glu Lys Thr Pro Val Ser Asp Arg Val Thr Lys Cys Cys Thr Glu Ser
465                 470                 475                 480

Leu Val Asn Arg Arg Pro Cys Phe Ser Ala Leu Glu Val Asp Glu Thr
                485                 490                 495

Tyr Val Pro Lys Glu Phe Asn Ala Glu Thr Phe Thr Phe His Ala Asp
            500                 505                 510

Ile Cys Thr Leu Ser Glu Lys Glu Arg Gln Ile Lys Lys Gln Thr Ala
        515                 520                 525

Leu Val Glu Leu Val Lys His Lys Pro Lys Ala Thr Lys Glu Gln Leu
    530                 535                 540

Lys Ala Val Met Asp Asp Phe Ala Ala Phe Val Glu Lys Cys Cys Lys
545                 550                 555                 560

Ala Asp Asp Lys Glu Thr Cys Phe Ala Glu Glu Gly Lys Lys Leu Val
                565                 570                 575

Ala Ala Ser Gln Ala Ala Leu Gly Leu
            580                 585

<210>  4
<211>  5
<212>  PRT
<213>  Artificial Sequence

<220>
<223>  Amino acid sequence of an exemplified linker 1

<400>  4

Gly Gly Gly Gly Ser
1               5

<210>  5
<211>  6
<212>  PRT
<213>  Artificial Sequence

<220>

<223>  Amino acid sequence of an exemplified linker 2

<400>  5

Gly Gly Gly Gly Gly Ser
1               5


<210>  6
<211>  5
<212>  PRT
<213>  Artificial Sequence

<220>
<223>  Amino acid sequence of an exemplified linker 3

<400>  6

Ser Gly Gly Gly Gly
1               5


<210>  7
<211>  23
<212>  DNA
<213>  Murine encephalomyocardinitis virus

<400>  7
atgataatat ggccacaacc atg                                               23


<210>  8
<211>  23
<212>  DNA
<213>  Artificial Sequence

<220>
<223>  Partial nucleotide sequence of IRES from mutant type murine
       encephalomyocardinitis virus

<400>  8
atgataagct tgccacaacc atg                                               23


<210>  9
<211>  191
<212>  PRT
<213>  Homo sapiens

<400>  9

<210>  10
<211>  176
<212>  PRT
<213>  Homo sapiens

<400>  10

Phe Pro Thr Ile Pro Leu Ser Arg Leu Phe Asp Asn Ala Met Leu Arg
1               5                   10                  15

Ala His Arg Leu His Gln Leu Ala Phe Asp Thr Tyr Gln Glu Phe Asn
            20                  25                  30

Pro Gln Thr Ser Leu Cys Phe Ser Glu Ser Ile Pro Thr Pro Ser Asn
        35                  40                  45

Arg Glu Glu Thr Gln Gln Lys Ser Asn Leu Glu Leu Leu Arg Ile Ser
    50                  55                  60

Leu Leu Leu Ile Gln Ser Trp Leu Glu Pro Val Gln Phe Leu Arg Ser
65                  70                  75                  80

Val Phe Ala Asn Ser Leu Val Tyr Gly Ala Ser Asp Ser Asn Val Tyr
                85                  90                  95

Asp Leu Leu Lys Asp Leu Glu Glu Gly Ile Gln Thr Leu Met Gly Arg
            100                 105                 110

Leu Glu Asp Gly Ser Pro Arg Thr Gly Gln Ile Phe Lys Gln Thr Tyr
        115                 120                 125

Ser Lys Phe Asp Thr Asn Ser His Asn Asp Asp Ala Leu Leu Lys Asn
    130                 135                 140

Tyr Gly Leu Leu Tyr Cys Phe Arg Lys Asp Met Asp Lys Val Glu Thr
145                 150                 155                 160

Phe Leu Arg Ile Val Gln Cys Arg Ser Val Glu Gly Ser Cys Gly Phe
                165                 170                 175

<210>  11
<211>  776
<212>  PRT
<213>  Artificial Sequence

<220>
<223>  Amino acid sequence of 22KhGH-mHSA

<400>  11

Phe Pro Thr Ile Pro Leu Ser Arg Leu Phe Asp Asn Ala Met Leu Arg
1               5                   10                  15

Ala His Arg Leu His Gln Leu Ala Phe Asp Thr Tyr Gln Glu Phe Glu
            20                  25                  30

Glu Ala Tyr Ile Pro Lys Glu Gln Lys Tyr Ser Phe Leu Gln Asn Pro
        35                  40                  45

Gln Thr Ser Leu Cys Phe Ser Glu Ser Ile Pro Thr Pro Ser Asn Arg
    50                  55                  60

Glu Glu Thr Gln Gln Lys Ser Asn Leu Glu Leu Leu Arg Ile Ser Leu
65                  70                  75                  80

Leu Leu Ile Gln Ser Trp Leu Glu Pro Val Gln Phe Leu Arg Ser Val
                85                  90                  95

Phe Ala Asn Ser Leu Val Tyr Gly Ala Ser Asp Ser Asn Val Tyr Asp
            100                 105                 110

Leu Leu Lys Asp Leu Glu Glu Gly Ile Gln Thr Leu Met Gly Arg Leu

115                 120                 125

Glu Asp Gly Ser Pro Arg Thr Gly Gln Ile Phe Lys Gln Thr Tyr Ser
    130                 135                 140

Lys Phe Asp Thr Asn Ser His Asn Asp Asp Ala Leu Leu Lys Asn Tyr
145                 150                 155                 160

Gly Leu Leu Tyr Cys Phe Arg Lys Asp Met Asp Lys Val Glu Thr Phe
                165                 170                 175

Leu Arg Ile Val Gln Cys Arg Ser Val Glu Gly Ser Cys Gly Phe Asp
            180                 185                 190

Ala His Lys Ser Glu Val Ala His Arg Phe Lys Asp Leu Gly Glu Glu
        195                 200                 205

Asn Phe Lys Ala Leu Val Leu Ile Ala Phe Ala Gln Tyr Leu Gln Gln
    210                 215                 220

Cys Pro Phe Glu Asp His Val Lys Leu Val Asn Glu Val Thr Glu Phe
225                 230                 235                 240

Ala Lys Thr Cys Val Ala Asp Glu Ser Ala Glu Asn Cys Asp Lys Ser
                245                 250                 255

Leu His Thr Leu Phe Gly Asp Lys Leu Cys Thr Val Ala Thr Leu Arg
            260                 265                 270

Glu Thr Tyr Gly Glu Met Ala Asp Cys Cys Ala Lys Gln Glu Pro Glu
        275                 280                 285

Arg Asn Glu Cys Phe Leu Gln His Lys Asp Asp Asn Pro Asn Leu Pro
    290                 295                 300

Arg Leu Val Arg Pro Glu Val Asp Val Met Cys Thr Ala Phe His Asp
305                 310                 315                 320

Asn Glu Glu Thr Phe Leu Lys Lys Tyr Leu Tyr Glu Ile Ala Arg Arg
                325                 330                 335

His Pro Tyr Phe Tyr Ala Pro Glu Leu Leu Phe Phe Ala Lys Arg Tyr
            340                 345                 350

Lys Ala Ala Phe Thr Glu Cys Cys Gln Ala Ala Asp Lys Ala Ala Cys
        355                 360                 365

Leu Leu Pro Lys Leu Asp Glu Leu Arg Asp Glu Gly Lys Ala Ser Ser
    370                 375                 380

Ala Lys Gln Arg Leu Lys Cys Ala Ser Leu Gln Lys Phe Gly Glu Arg
385                 390                 395                 400

Ala Phe Lys Ala Trp Ala Val Ala Arg Leu Ser Gln Arg Phe Pro Lys
                405                 410                 415

Ala Glu Phe Ala Glu Val Ser Lys Leu Val Thr Asp Leu Thr Lys Val
            420                 425                 430

His Thr Glu Cys Cys His Gly Asp Leu Leu Glu Cys Ala Asp Asp Arg
        435                 440                 445

Ala Asp Leu Ala Lys Tyr Ile Cys Glu Asn Gln Asp Ser Ile Ser Ser
    450                 455                 460

Lys Leu Lys Glu Cys Cys Glu Lys Pro Leu Leu Glu Lys Ser His Cys
465                 470                 475                 480

Ile Ala Glu Val Glu Asn Asp Glu Met Pro Ala Asp Leu Pro Ser Leu
                485                 490                 495

Ala Ala Asp Phe Val Glu Ser Lys Asp Val Cys Lys Asn Tyr Thr Glu
            500                 505                 510

Ala Lys Asp Val Phe Leu Gly Met Phe Leu Tyr Glu Tyr Ala Arg Arg
        515                 520                 525

His Pro Asp Tyr Ser Val Val Leu Leu Leu Arg Leu Ala Lys Thr Tyr
    530                 535                 540

Glu Thr Thr Leu Glu Lys Cys Cys Ala Ala Ala Asp Pro His Glu Cys
545                 550                 555                 560

Tyr Ala Lys Val Phe Asp Glu Phe Lys Pro Leu Val Glu Glu Pro Gln
                565                 570                 575

Asn Leu Ile Lys Gln Asn Cys Glu Leu Phe Glu Gln Leu Gly Glu Tyr
            580                 585                 590

Lys Phe Gln Asn Ala Leu Leu Val Arg Tyr Thr Lys Lys Val Pro Gln
        595                 600                 605

Val Ser Thr Pro Thr Leu Val Glu Val Ser Arg Asn Leu Gly Lys Val
    610                 615                 620

Gly Ser Lys Cys Cys Lys His Pro Glu Ala Lys Arg Met Pro Cys Ala
625                 630                 635                 640

Glu Asp Tyr Leu Ser Val Val Leu Asn Gln Leu Cys Val Leu His Glu
                645                 650                 655

Lys Thr Pro Val Ser Asp Arg Val Thr Lys Cys Cys Thr Glu Ser Leu
            660                 665                 670

Val Asn Arg Arg Pro Cys Phe Ser Ala Leu Glu Val Asp Glu Thr Tyr
        675                 680                 685

Val Pro Lys Glu Phe Asn Ala Glu Thr Phe Thr Phe His Ala Asp Ile
    690                 695                 700

Cys Thr Leu Ser Glu Lys Glu Arg Gln Ile Lys Lys Gln Thr Ala Leu
705                 710                 715                 720

Val Glu Leu Val Lys His Lys Pro Lys Ala Thr Lys Glu Gln Leu Lys
                725                 730                 735

Ala Val Met Asp Asp Phe Ala Ala Phe Val Glu Lys Cys Cys Lys Ala
            740                 745                 750

Asp Asp Lys Glu Thr Cys Phe Ala Glu Glu Gly Lys Lys Leu Val Ala
        755                 760                 765

Ala Ser Gln Ala Ala Leu Gly Leu
    770                 775

<210>  12
<211>  761
<212>  PRT
<213>  Artificial Sequence

<220>
<223>  Amino acid sequence of 20KhGH-mHSA

<400>  12

Phe Pro Thr Ile Pro Leu Ser Arg Leu Phe Asp Asn Ala Met Leu Arg
1               5                   10                  15

Ala His Arg Leu His Gln Leu Ala Phe Asp Thr Tyr Gln Glu Phe Asn
            20                  25                  30

Pro Gln Thr Ser Leu Cys Phe Ser Glu Ser Ile Pro Thr Pro Ser Asn
        35                  40                  45

Arg Glu Glu Thr Gln Gln Lys Ser Asn Leu Glu Leu Leu Arg Ile Ser
    50                  55                  60

Leu Leu Leu Ile Gln Ser Trp Leu Glu Pro Val Gln Phe Leu Arg Ser
65                  70                  75                  80

Val Phe Ala Asn Ser Leu Val Tyr Gly Ala Ser Asp Ser Asn Val Tyr
                85                  90                  95

Asp Leu Leu Lys Asp Leu Glu Glu Gly Ile Gln Thr Leu Met Gly Arg
            100                 105                 110

Leu Glu Asp Gly Ser Pro Arg Thr Gly Gln Ile Phe Lys Gln Thr Tyr
        115                 120                 125

Ser Lys Phe Asp Thr Asn Ser His Asn Asp Asp Ala Leu Leu Lys Asn
    130                 135                 140

Tyr Gly Leu Leu Tyr Cys Phe Arg Lys Asp Met Asp Lys Val Glu Thr
145                 150                 155                 160

Phe Leu Arg Ile Val Gln Cys Arg Ser Val Glu Gly Ser Cys Gly Phe
                165                 170                 175

Asp Ala His Lys Ser Glu Val Ala His Arg Phe Lys Asp Leu Gly Glu
            180                 185                 190

Glu Asn Phe Lys Ala Leu Val Leu Ile Ala Phe Ala Gln Tyr Leu Gln
        195                 200                 205

Gln Cys Pro Phe Glu Asp His Val Lys Leu Val Asn Glu Val Thr Glu
    210                 215                 220

Phe Ala Lys Thr Cys Val Ala Asp Glu Ser Ala Glu Asn Cys Asp Lys
225                 230                 235                 240

Ser Leu His Thr Leu Phe Gly Asp Lys Leu Cys Thr Val Ala Thr Leu
                245                 250                 255

Arg Glu Thr Tyr Gly Glu Met Ala Asp Cys Cys Ala Lys Gln Glu Pro
            260                 265                 270

Glu Arg Asn Glu Cys Phe Leu Gln His Lys Asp Asp Asn Pro Asn Leu
        275                 280                 285

Pro Arg Leu Val Arg Pro Glu Val Asp Val Met Cys Thr Ala Phe His
    290                 295                 300

Asp Asn Glu Glu Thr Phe Leu Lys Lys Tyr Leu Tyr Glu Ile Ala Arg
305                 310                 315                 320

Arg His Pro Tyr Phe Tyr Ala Pro Glu Leu Leu Phe Phe Ala Lys Arg
                325                 330                 335

Tyr Lys Ala Ala Phe Thr Glu Cys Cys Gln Ala Ala Asp Lys Ala Ala
            340                 345                 350

Cys Leu Leu Pro Lys Leu Asp Glu Leu Arg Asp Glu Gly Lys Ala Ser
        355                 360                 365

Ser Ala Lys Gln Arg Leu Lys Cys Ala Ser Leu Gln Lys Phe Gly Glu
    370                 375                 380

Arg Ala Phe Lys Ala Trp Ala Val Ala Arg Leu Ser Gln Arg Phe Pro
385                 390                 395                 400

Lys Ala Glu Phe Ala Glu Val Ser Lys Leu Val Thr Asp Leu Thr Lys
                405                 410                 415

Val His Thr Glu Cys Cys His Gly Asp Leu Leu Glu Cys Ala Asp Asp
            420                 425                 430

Arg Ala Asp Leu Ala Lys Tyr Ile Cys Glu Asn Gln Asp Ser Ile Ser
        435                 440                 445

Ser Lys Leu Lys Glu Cys Cys Glu Lys Pro Leu Leu Glu Lys Ser His
    450                 455                 460

Cys Ile Ala Glu Val Glu Asn Asp Glu Met Pro Ala Asp Leu Pro Ser
465                 470                 475                 480

Leu Ala Ala Asp Phe Val Glu Ser Lys Asp Val Cys Lys Asn Tyr Thr
                485                 490                 495

Glu Ala Lys Asp Val Phe Leu Gly Met Phe Leu Tyr Glu Tyr Ala Arg
            500                 505                 510

Arg His Pro Asp Tyr Ser Val Val Leu Leu Leu Arg Leu Ala Lys Thr
        515                 520                 525

Tyr Glu Thr Thr Leu Glu Lys Cys Cys Ala Ala Ala Asp Pro His Glu
    530                 535                 540

Cys Tyr Ala Lys Val Phe Asp Glu Phe Lys Pro Leu Val Glu Glu Pro
545                 550                 555                 560

Gln Asn Leu Ile Lys Gln Asn Cys Glu Leu Phe Glu Gln Leu Gly Glu
                565                 570                 575

Tyr Lys Phe Gln Asn Ala Leu Leu Val Arg Tyr Thr Lys Lys Val Pro
            580                 585                 590

Gln Val Ser Thr Pro Thr Leu Val Glu Val Ser Arg Asn Leu Gly Lys
        595                 600                 605

Val Gly Ser Lys Cys Cys Lys His Pro Glu Ala Lys Arg Met Pro Cys
    610                 615                 620

Ala Glu Asp Tyr Leu Ser Val Val Leu Asn Gln Leu Cys Val Leu His
625                 630                 635                 640

Glu Lys Thr Pro Val Ser Asp Arg Val Thr Lys Cys Cys Thr Glu Ser
                645                 650                 655

Leu Val Asn Arg Arg Pro Cys Phe Ser Ala Leu Glu Val Asp Glu Thr
            660                 665                 670

Tyr Val Pro Lys Glu Phe Asn Ala Glu Thr Phe Thr Phe His Ala Asp
        675                 680                 685

Ile Cys Thr Leu Ser Glu Lys Glu Arg Gln Ile Lys Lys Gln Thr Ala
    690                 695                 700

Leu Val Glu Leu Val Lys His Lys Pro Lys Ala Thr Lys Glu Gln Leu
705                 710                 715                 720

Lys Ala Val Met Asp Asp Phe Ala Ala Phe Val Glu Lys Cys Cys Lys
                725                 730                 735

Ala Asp Asp Lys Glu Thr Cys Phe Ala Glu Glu Gly Lys Lys Leu Val
            740                 745                 750

Ala Ala Ser Gln Ala Ala Leu Gly Leu
        755                 760

<210>  13
<211>  27
<212>  DNA
<213>  Artificial Sequence

<220>
<223>  Primer YA082, synthetic sequence

<400>  13

aactatactg aggcaaagga tgtcttc                                          27

<210>  14
<211>  27
<212>  DNA
<213>  Artificial Sequence

<220>
<223>  Primer YA083, synthetic sequence

<400>  14
tgcctcagta tagtttttgc aaacatc                                          27

<210>  15
<211>  776
<212>  PRT
<213>  Artificial Sequence

<220>
<223>  Amino acid sequence of 22KhGH-HSA

<400>  15

Phe Pro Thr Ile Pro Leu Ser Arg Leu Phe Asp Asn Ala Met Leu Arg
1               5                   10                  15

Ala His Arg Leu His Gln Leu Ala Phe Asp Thr Tyr Gln Glu Phe Glu
            20                  25                  30

Glu Ala Tyr Ile Pro Lys Glu Gln Lys Tyr Ser Phe Leu Gln Asn Pro
        35                  40                  45

Gln Thr Ser Leu Cys Phe Ser Glu Ser Ile Pro Thr Pro Ser Asn Arg
    50                  55                  60

Glu Glu Thr Gln Gln Lys Ser Asn Leu Glu Leu Leu Arg Ile Ser Leu
65                  70                  75                  80

Leu Leu Ile Gln Ser Trp Leu Glu Pro Val Gln Phe Leu Arg Ser Val
                85                  90                  95

Phe Ala Asn Ser Leu Val Tyr Gly Ala Ser Asp Ser Asn Val Tyr Asp
            100                 105                 110

Leu Leu Lys Asp Leu Glu Glu Gly Ile Gln Thr Leu Met Gly Arg Leu
        115                 120                 125

Glu Asp Gly Ser Pro Arg Thr Gly Gln Ile Phe Lys Gln Thr Tyr Ser
    130                 135                 140

Lys Phe Asp Thr Asn Ser His Asn Asp Asp Ala Leu Leu Lys Asn Tyr
145                 150                 155                 160

Gly Leu Leu Tyr Cys Phe Arg Lys Asp Met Asp Lys Val Glu Thr Phe
                165                 170                 175

Leu Arg Ile Val Gln Cys Arg Ser Val Glu Gly Ser Cys Gly Phe Asp
            180                 185                 190

Ala His Lys Ser Glu Val Ala His Arg Phe Lys Asp Leu Gly Glu Glu
        195                 200                 205

Asn Phe Lys Ala Leu Val Leu Ile Ala Phe Ala Gln Tyr Leu Gln Gln
    210                 215                 220

Cys Pro Phe Glu Asp His Val Lys Leu Val Asn Glu Val Thr Glu Phe
225                 230                 235                 240

Ala Lys Thr Cys Val Ala Asp Glu Ser Ala Glu Asn Cys Asp Lys Ser
                245                 250                 255

Leu His Thr Leu Phe Gly Asp Lys Leu Cys Thr Val Ala Thr Leu Arg
            260                 265                 270

Glu Thr Tyr Gly Glu Met Ala Asp Cys Cys Ala Lys Gln Glu Pro Glu
        275                 280                 285

Arg Asn Glu Cys Phe Leu Gln His Lys Asp Asp Asn Pro Asn Leu Pro
    290                 295                 300

Arg Leu Val Arg Pro Glu Val Asp Val Met Cys Thr Ala Phe His Asp
305                 310                 315                 320

Asn Glu Glu Thr Phe Leu Lys Lys Tyr Leu Tyr Glu Ile Ala Arg Arg
                325                 330                 335

His Pro Tyr Phe Tyr Ala Pro Glu Leu Leu Phe Phe Ala Lys Arg Tyr
            340                 345                 350

Lys Ala Ala Phe Thr Glu Cys Cys Gln Ala Ala Asp Lys Ala Ala Cys
        355                 360                 365

Leu Leu Pro Lys Leu Asp Glu Leu Arg Asp Glu Gly Lys Ala Ser Ser
    370                 375                 380

Ala Lys Gln Arg Leu Lys Cys Ala Ser Leu Gln Lys Phe Gly Glu Arg
385                 390                 395                 400

Ala Phe Lys Ala Trp Ala Val Ala Arg Leu Ser Gln Arg Phe Pro Lys

                405                 410                 415

Ala Glu Phe Ala Glu Val Ser Lys Leu Val Thr Asp Leu Thr Lys Val
            420                 425                 430

His Thr Glu Cys Cys His Gly Asp Leu Leu Glu Cys Ala Asp Asp Arg
        435                 440                 445

Ala Asp Leu Ala Lys Tyr Ile Cys Glu Asn Gln Asp Ser Ile Ser Ser
    450                 455                 460

Lys Leu Lys Glu Cys Cys Glu Lys Pro Leu Leu Glu Lys Ser His Cys
465                 470                 475                 480

Ile Ala Glu Val Glu Asn Asp Glu Met Pro Ala Asp Leu Pro Ser Leu
                485                 490                 495

Ala Ala Asp Phe Val Glu Ser Lys Asp Val Cys Lys Asn Tyr Ala Glu
            500                 505                 510

Ala Lys Asp Val Phe Leu Gly Met Phe Leu Tyr Glu Tyr Ala Arg Arg
        515                 520                 525

His Pro Asp Tyr Ser Val Val Leu Leu Leu Arg Leu Ala Lys Thr Tyr
    530                 535                 540

Glu Thr Thr Leu Glu Lys Cys Cys Ala Ala Ala Asp Pro His Glu Cys
545                 550                 555                 560

Tyr Ala Lys Val Phe Asp Glu Phe Lys Pro Leu Val Glu Glu Pro Gln
                565                 570                 575

Asn Leu Ile Lys Gln Asn Cys Glu Leu Phe Glu Gln Leu Gly Glu Tyr
            580                 585                 590

Lys Phe Gln Asn Ala Leu Leu Val Arg Tyr Thr Lys Lys Val Pro Gln
        595                 600                 605

Val Ser Thr Pro Thr Leu Val Glu Val Ser Arg Asn Leu Gly Lys Val
    610                 615                 620

Gly Ser Lys Cys Cys Lys His Pro Glu Ala Lys Arg Met Pro Cys Ala
625                 630                 635                 640

Glu Asp Tyr Leu Ser Val Val Leu Asn Gln Leu Cys Val Leu His Glu
                645                 650                 655

Lys Thr Pro Val Ser Asp Arg Val Thr Lys Cys Cys Thr Glu Ser Leu
            660                 665                 670

Val Asn Arg Arg Pro Cys Phe Ser Ala Leu Glu Val Asp Glu Thr Tyr
        675                 680                 685

Val Pro Lys Glu Phe Asn Ala Glu Thr Phe Thr Phe His Ala Asp Ile
    690                 695                 700

Cys Thr Leu Ser Glu Lys Glu Arg Gln Ile Lys Lys Gln Thr Ala Leu
705                 710                 715                 720

Val Glu Leu Val Lys His Lys Pro Lys Ala Thr Lys Glu Gln Leu Lys
                725                 730                 735

Ala Val Met Asp Asp Phe Ala Ala Phe Val Glu Lys Cys Cys Lys Ala
            740                 745                 750

Asp Asp Lys Glu Thr Cys Phe Ala Glu Glu Gly Lys Lys Leu Val Ala
        755                 760                 765

Ala Ser Gln Ala Ala Leu Gly Leu
    770                 775

<210>  16
<211>  2427
<212>  DNA
<213>  Artificial Sequence

<220>
<223>  Nucleotide sequence containing 22KhGH-HSA gene, synthetic sequence

<400>  16
acgcgtcacc atggctacag gctcccggac gtccctgctc ctggcttttg gcctgctctg      60

cctgccctgg cttcaagagg gcagtgcctt cccaaccatt cccttatcca ggctttttga     120

caacgctatg ctccgcgccc atcgtctgca ccagctggcc tttgacacct accaggagtt     180

tgaagaagcc tatatcccaa aggaacagaa gtattcattc ctgcagaacc cccagacctc     240

cctctgtttc tcagagtcta ttccgacacc ctccaacagg gaggaaacac aacagaaatc     300

caacctagag ctgctccgca tctccctgct gctcatccag tcgtggctgg agcccgtgca     360

gttcctcagg agtgtcttcg ccaacagcct ggtgtacggc gcctctgaca gcaacgtcta     420

tgacctccta aaggacctag aggaaggcat ccaaacgctg atggggaggc tggaagatgg     480

cagcccccgg actgggcaga tcttcaagca gacctacagc aagttcgaca caaactcaca     540

caacgatgac gcactactca agaactacgg gctgctctac tgcttcagga aggacatgga     600

caaggtcgag acattcctgc gcatcgtgca gtgccgctct gtggagggca gctgtggctt     660

cgatgcacac aagagtgagg ttgctcatcg gtttaaagat ttgggagaag aaaatttcaa 720

agccttggtg ttgattgcct ttgctcagta tcttcagcag tgtccatttg aagatcatgt 780

aaaattagtg aatgaagtaa ctgaatttgc aaaaacatgt gttgctgatg agtcagctga 840

aaattgtgac aaatcacttc ataccctttt tggagacaaa ttatgcacag ttgcaactct 900

tcgtgaaacc tatggtgaaa tggctgactg ctgtgcaaaa caagaacctg agagaaatga 960

atgcttcttg caacacaaag atgacaaccc aaacctcccc cgattggtga gaccagaggt 1020

tgatgtgatg tgcactgctt ttcatgacaa tgaagagaca tttttgaaaa aatacttata 1080

tgaaattgcc agaagacatc cttactttta tgccccggaa ctccttttct ttgctaaaag 1140

gtataaagct gcttttacag aatgttgcca agctgctgat aaagctgcct gcctgttgcc 1200

aaagctcgat gaacttcggg atgaagggaa ggcttcgtct gccaaacaga gactcaagtg 1260

tgccagtctc caaaaatttg gagaaagagc tttcaaagca tgggcagtag ctcgcctgag 1320

ccagagattt cccaaagctg agtttgcaga agtttccaag ttagtgacag atcttaccaa 1380

agtccacacg gaatgctgcc atggagatct gcttgaatgt gctgatgaca gggcggacct 1440

tgccaagtat atctgtgaaa atcaagattc gatctccagt aaactgaagg aatgctgtga 1500

aaaacctctg ttggaaaaat cccactgcat tgccgaagtg gaaaatgatg agatgcctgc 1560

tgacttgcct tcattagctg ctgattttgt tgaaagtaag gatgtttgca aaaactatgc 1620

tgaggcaaag gatgtcttcc tgggcatgtt tttgtatgaa tatgcaagaa ggcatcctga 1680

ttactctgtc gtgctgctgc tgagacttgc caagacatat gaaaccactc tagagaagtg 1740

ctgtgccgct gcagatcctc atgaatgcta tgccaaagtg ttcgatgaat ttaaacctct 1800

tgtggaagag cctcagaatt taatcaaaca aaattgtgag ctttttgagc agcttggaga 1860

gtacaaattc cagaatgcgc tattagttcg ttacaccaag aaagtacccc aagtgtcaac 1920

tccaactctt gtagaggtct caagaaacct aggaaaagtg ggcagcaaat gttgtaaaca 1980

tcctgaagca aaaagaatgc cctgtgcaga agactatcta tccgtggtcc tgaaccagtt 2040

atgtgtgttg catgagaaaa cgccagtaag tgacagagtc accaaatgct gcacagaatc 2100

cttggtgaac aggcgaccat gcttttcagc tctggaagtc gatgaaacat acgttcccaa 2160

agagtttaat gctgaaacat tcaccttcca tgcagatata tgcacacttt ctgagaagga 2220

gagacaaatc aagaaacaaa ctgcacttgt tgagctcgtg aaacacaagc ccaaggcaac 2280

aaaagagcaa ctgaaagctg ttatggatga tttcgcagct tttgtagaga agtgctgcaa 2340

ggctgacgat aaggagacct gctttgccga ggagggtaaa aaacttgttg ctgcaagtca 2400

agctgcctta ggcttataag cggccgc 2427